(19) Europäisches Patentamt
European Patent Office
Office européen des brevets

(11) **EP 2 472 264 B1**

(12) **EUROPEAN PATENT SPECIFICATION**

(45) Date of publication and mention
of the grant of the patent:
**25.11.2015 Bulletin 2015/48**

(51) Int Cl.:
***G01N 33/574*** *(2006.01)*

(21) Application number: **12002007.8**

(22) Date of filing: **27.02.2008**

(54) **MULTIPLEX DETECTION OF TUMOUR CELLS USING A PANEL OF AGENTS BINDING TO EXTRACELLULAR MARKERS**

MULTIPLEX-NACHWEIS VON TUMORZELLEN UNTER VERWENDUNG EINER GRUPPE VON EXTRAZELLULÄRE MARKER BINDENDEN AGENTEN

DÉTECTION MULTIPLEX DE CELLULES TUMORALES UTILISANT PLUSIEURS AGENTS SE FIXANT À DES MARQUEURS EXTRACELLULAIRES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.02.2007 US 903986 P**

(43) Date of publication of application:
**04.07.2012 Bulletin 2012/27**

(62) Document number(s) of the earlier application(s) in accordance with Art. 76 EPC:
**08716076.8 / 2 126 579**

(73) Proprietor: **SentoClone International AB**
**171 65 Solna (SE)**

(72) Inventors:
• **Winqvist, Ola**
**756 53 Uppsala (SE)**
• **Thorn, Magnus**
**752 26 Uppsala (SE)**

(74) Representative: **Schöneborn, Holger**
**Schneiders & Behrendt**
**Rechts- und Patentanwälte**
**Huestraße 23**
**(Westfalenbankgebäude)**
**44787 Bochum (DE)**

(56) References cited:
**WO-A1-02/20825          WO-A1-98/28622**
**WO-A2-2008/008515     US-A1- 2003 129 676**

• **DURRANT L G ET AL: "ENHANCED RECOGNITION OF HUMAN COLORECTAL TUMOR CELLS USING COMBINATIONS OF MONOCLONAL ANTIBODIES", BRITISH JOURNAL OF CANCER, vol. 60, no. 6, 1989, pages 855-860, XP008078261, ISSN: 0007-0920**
• **ANDREW S M ET AL: "TUMOR LOCALIZATION BY COMBINATIONS OF MONOCLONAL ANTIBODIES IN A NEW HUMAN COLON CARCINOMA CELL LINE (LIM1899)", CANCER RESEARCH, AMERICAN ASSOCIATION FOR CANCER RESEARCH, BALTIMORE, MD, US, vol. 50, no. 17, 1 September 1990 (1990-09-01), pages 5225-5230, XP001246404, ISSN: 0008-5472**
• **O'BRIEN CATHERINE A ET AL: "A human colon cancer cell capable of initiating tumour growth in immunodeficient mice", NATURE (LONDON), vol. 445, no. 7123, January 2007 (2007-01) , pages 106-110, XP002431334, ISSN: 0028-0836**
• **MOLNAR BELA ET AL: "Molecular detection of circulating cancer cells. Role in diagnosis, prognosis and follow-up of colon cancer patients.", DIGESTIVE DISEASES, vol. 21, no. 4, 2003, pages 320-325, XP008078262, ISSN: 0257-2753**
• **MEHES G ET AL: "Detecting disseminated solid tumor cells in hematopoietic samples: Methodological aspects", HAEMATOLOGIA, vol. 31, no. 2, 2001, pages 97-109, XP008078323, ISSN: 0017-6559**

EP 2 472 264 B1

- PACKEISEN J ET AL: "DETECTION OF SURFACE ANTIGEN 17-1A IN BREAST AND COLORECTAL CANCER", HYBRIDOMA, LIEBERT, NEW YORK, NY, US, vol. 18, no. 1, February 1999 (1999-02), pages 37-40, XP009016649, ISSN: 0272-457X
- MARITS P ET AL: "Detection of Immune Responses Against Urinary Bladder Cancer in Sentinel Lymph Nodes", EUROPEAN UROLOGY, ELSEVIER BV, NL, vol. 49, no. 1, 1 January 2006 (2006-01-01), pages 59-70, XP027882341, ISSN: 0302-2838 [retrieved on 2006-01-01]
- Frank Momburg: "Immunohistochemical Study of the Expression of a Mr 34,000 Human Epithelium-specific Surface Glycoprotein in Normal and Malignant Tissues", Cancer Research, 1 June 1987 (1987-06-01), pages 2883-2891, XP55036979, Retrieved from the Internet: URL:http://cancerres.aacrjournals.org/cont ent/47/11/2883.full.pdf [retrieved on 2012-09-03]
- John Magnani: "A Monoclonal Antibody-defined Antigen Associated with Gastrointestinal Cancer Is a Ganglioside Containing Sialylated Lacto-N-fucopentaose 11*", The Journal of Biological Chemistry, 10 December 1982 (1982-12-10), pages 14365-14369, XP55036982, Retrieved from the Internet: URL:http://www.jbc.org/content/257/23/1436 5.full.pdf [retrieved on 2012-09-03]
- KARLSSON M ET AL: "Detection of metastatic colon cancer cells in sentinel nodes by flow cytometry", JOURNAL OF IMMUNOLOGICAL METHODS, ELSEVIER SCIENCE PUBLISHERS B.V.,AMSTERDAM, NL, vol. 334, no. 1-2, 20 May 2008 (2008-05-20), pages 122-133, XP022614840, ISSN: 0022-1759, DOI: 10.1016/J.JIM.2008.02.008 [retrieved on 2008-03-10]

**Description**

**Field of the invention**

The present invention relates to a method for detection of cancer cell or cancer cell elements in a biological sample, wherein the method comprises addition of a panel of agents which bind to three or more extracellular markers and wherein at least one agent is cancer specific. The detection is carried out by a spectro-metric method.

**Background of the invention**

Studies of prognostic factors of cancers identify the histological status of the regional lymph nodes as one of the most important predictors of patient survival. The sentinel node concept, first introduced by Raymond Cabanas (Cabanas et al., 1977), proposes that the lymphatic drainage from a primary tumour passes through one particular regional lymph node - the sentinel node - and then continues. The sentinel node concept entails the possibility of multiple drainage pathways from different parts of the primary tumour, thus a patient can have more than one sentinel node. The sentinel node is unique for each individual. The tumour status of this node reflects the status of the regional lymphatic field and has a strong impact on prognosis, as reported in large follow-up studies of malignant melanoma and breast cancer.

It has been reported that the 5-year survival rate is as high as 89% for melanoma patients diagnosed with a tumour cell metastasis negative sentinel node. For patients diagnosed with metastasized sentinel node, the 5-year survival is reduced to 70% (Morton et al., 2003). It can be speculated that the 11 % deceased in the group diagnosed with metastases free sentinel nodes were in fact false-negative diagnosed cases.

Due to the strong evidence of sentinel node status as a prognostic factor, sentinel node biopsy and eventual evaluation of presence or absence of metastatic tumour cells has become routine procedure for staging in many countries.

The diagnosis of the sentinel node also has impact on the treatment strategy selected once the sentinel node is diagnosed. If sentinel node status can be rapidly determined it can also provide valuable information for decision-making during surgery.

Today, the routine procedure of sentinel lymph node evaluation is postoperative histopathological examination of formalin-fixed paraffin-embedded sections from several levels of the node. However, micrometastases located between the sections may be missed. One further development is intraoperative investigation of the sentinel node. In this method, thin frozen sections (about 5 $\mu$m thick) of the lymph node are stained with hematoxylin and eosin. The frozen sections are then examined under the microscope by a pathologist. The result is reported back to the operating theatre and decisions can be made to for example extend the operation with lymph node dissection if the node contains tumour cells. Postoperatively, the specimens are sometimes also immunohistochemically stained for markers specific for the cancer type examined, which facilitates detection of micrometastases. Examples of markers targeted are HMB-45 for malignant melanoma and cytokeratin-20 for colon and breast cancer.

These routine procedures of tumour cell detection suffer from various shortcomings. They are time consuming, reducing the possibility of providing important information during primary tumour surgery. Worst case, it is also inaccurate as only a small fraction (very thin slices) of the lymph node is examined and micro-metastases outside these slices will inevitably not be detected. The probability of detecting a metastasis using routine screening of cytokeratin-20 stained frozen sections is also dependent upon the size of the metastasis. In a specific study with breast cancer sentinel nodes (Weaver et al., 2006), the probability of missing a metastasis $\leq$ 0.10 mm was found to be 61 %. For metastases $\leq$ 0.05 mm and $\leq$ 0.02 the probability of missing the presence of tumour cells was 69% and 75%, respectively. The method also relies upon the individual skill of the pathologist to detect tumour cells. A final common problem with the current method is shortage of personnel skilled in the art at hospital laboratories, which has implications on the availability and cost of these procedures.

Alternative methodologies of detection have also been reported, including reverse transcriptase polymerase chain reaction (RT-PCR) and flow-cytometric approaches. The RT-PCR methodology is more sensitive than the histopathological method, but it is also time consuming and the method has been found to produce false-positive results (Merrie et al., 1999; Noguchi et al., 1999). In addition, mRNA is sensitive to degradation, a feature making handling and transport of the specimen difficult in a routine clinical setting.

Flow-cytometric approaches have been reported to possess higher sensitivity than conventional techniques (Leers et al., 2002). So far, methods involving flow cytometry as a tool of detecting tumour cells in lymph nodes have relied on staining with monoclonal antibodies against intracellular markers such as cytokeratin-20, a protein produced by various cancer cells (e.g. breast and colon cancer), and DNA.

Relying upon cytokeratin-20 implies a risk of a false-positive diagnosis, as this protein is not exclusively expressed by tumour cells. Experiments have shown that this protein is also expressed in cells present in peripheral blood of healthy subjects. Accordingly, a need arise to determine a "cut-off" rate between a positive and negative diagnosis, which inevitably carries with it a risk of false results.

Using intracellular markers like cytokeratin-20 or HMB-45 also requires an extra step in the antibody staining procedure. Cells must be permeabilised with e.g. saponin before monoclonal antibodies can reach their targets adding at least 30 minutes to the procedure.

A flow cytometry based method is also known from WO 98/28622 A1, wherein several markers are provided, including extracellular markers like CEA and CA15-3.

Furthermore, the expression pattern varies dramatically between tumour cells and using only a single marker implies a risk of producing false-negative results.

Starting from the described prior art it was the purpose of the present invention to provide a fast, robust, standardised analytical method based upon non-subjective physical characteristics with high specificity, sensitivity and reproducibility.

**Description of the invention**

The present invention relates to a one step method for direct and simultaneous detection of a cancer cell or cancer cell element in a biological sample, namely tumour cell detection in sentinel nodes, metinel nodes, or other lymph nodes, the method comprising

i) adding to said biological sample a panel of labelled agents, which bind to three or more different extracellular markers, wherein at least one of the agents is cancer specific and wherein the panel contains:

- one or more agents binding to cancer-type specific extracellular markers, wherein the cancer-type specific marker is CA19-9, and
- one or more agents binding to tissue-specific extracellular markers that are general epithelial extracellular markers, wherein the general epithelial marker is Ep-CAM;

ii) subjecting the thus obtained biological sample to flow cytometry.

The present invention relates to a method for detection of cancer cell or cancer cell elements in a biological example, wherein the method comprises addition of a panel of agents which bind to three or more extracellular markers and wherein at least one agent is cancer specific. The detection is carried out by a spectrometric method. The present invention thus encompasses a one step process by the use of a panel of agents which is added to said biological sample and thereby permitting direct simultaneous detection of several cancer specific markers, thus omitting the step of sentinel node dissection, staining of the dissection sample and the subsequent inspection by an experienced pathologist. As a consequence the present invention permits a considerably faster, robust, standardised analytical method based upon non-subjective physical characteristics with higher specificity, sensitivity and reproducibility.

The present invention has various applications. The method is used for tumour cell detection in sentinel nodes, metinel nodes, or other lymph nodes. As described above, tumour cell detection in sentinel nodes is a common procedure for cancer staging. Applying a panel of agents binding to extracellular markers that identify a certain tumour type allows for tumour cell detection with a very high sensitivity and specificity. A similar method can be applied for detection of cancer cells also in other tissues.

In a second diagnostic application, the present invention can be used for determining type of primary tumour in patients with metastases of unknown origin. By applying a panel or panels of agents binding to extracellular markers that are specific for certain types of cancer and/or types of tissue, the method can provide valuable clues in the search for the primary tumour site.

In yet another application of the present invention, the method can be used for monitoring of cell cultures for cell therapy against cancer (so called adoptive immunotherapy). The use of sentinel node lymphocytes in adoptive immunotherapy requires the need for detection of cancer cells in a single cell suspension In this application, a panel of agents binding to extracellular markers identifying the cancer type that is being treated could be used in order to verify that the cancer cells are absent from the cell culture before administering cells back to the patient. This application may also be used for the monitoring of patients during treatment and may be used as a tool to evaluate the effectiveness of a treatment regimen as well as a decisive tool for selection of appropriate therapy.

Usage of the present invention for tumour cell detection applications can be achieved by preparing a single cell suspension from different types of tissue. In a following step, the single cell suspension or a sample there from is incubated with a panel of fluorophore conjugated monoclonal antibodies binding to markers that identify the cancer type that should be detected. After incubation time and washing, the sample is analysed using flow cytometry and the results of the analysis are readily available.

As mentioned, the single cell suspension can be prepared from various sources, namely sentinel nodes, metinel nodes, or other lymph nodes.

In the second diagnostic application, identification of primary tumour, a single cell suspension is prepared from either a lymph node draining the metastasis of unknown origin or from a biopsy of the metastasis. Depending upon where in the body the metastasis has been found, several panels of fluorophore conjugated monoclonal antibodies can be used to identify specific primary tumour types that are frequently metastasised to the organ or tissue where the metastasis has been found. The single cell suspension may need to be divided in several samples, all incubated with different panels of antibodies, in order to screen for the tumour type that has metastasised. Analysis using flow cytometry is possible also in this application of the present invention.

Verification of absence of tumour cells in cell cultures for cell therapy against cancer can be achieved using a similar method as detection of tumour cells in lymph nodes. In this application of the present invention, the single cell suspension is prepared from a sample of the cell culture. Using the same type of panel of monoclonal antibodies as for the application of tumour cell detection, the single cell suspension is incubated, washed and finally analysed using flow cytometry.

*Definitions*

The term "agents" as used herein refers to all agents capable of binding or interacting with an extracellular marker, covering both already known agents, such as, e.g. commercially available monoclonal antibodies, and agents to be generated. Examples of suitable agents are monoclonal antibodies, polyclonal antibodies, Fab fragments, (Fab')$_2$ fragments, Fv fragments, peptides, proteins, RNA molecules, polysaccharides, or any combination thereof.

By the term "panel of agents" Is intended to mean a panel containing two or more agents, such as, e.g., three or more, four or more, five or more, six or more, seven or more, eight or more agents, nine or more, or ten or more agents- The number of agents chosen may depend on the number of markers to be screened for and/or the capacity of the equipment used in the method for quantifying the number of individual cells, i.e. how many agents the equipment is capable of detecting simultaneously. The number of cancer specific agents in the panel depends on the specific use, however, one or more cancer specific agents are present, such as, e.g., two or more, three or more, four or more, five or more, six or more, seven or more, eight or more agents, nine or more, or ten or more agents. Preferably much more specific agents are employed such as e.g. at least 20, at least 30, at least 40, at least 50, at least 75 or at least 100 dependent on the availability of the cancer specific agents. The cancer specific agents in a panel may all be specific for one type of cancer or may have specificities for different cancers types.

The term "antibodies" as used herein refers to all types of immunoglobulins, including IgG, IgM, IgA, IgD, IgE, and IgY. The antibodies may be monoclonal or polygonal and may be of any species of origin, including (for example) mouse, rat, rabbit, horse, chicken, or human, or may be chimeric antibodies. Polyclonal antibodies used to carry out the present invention may be produced by immunizing a suitable animal (e.g., rabbit, goat, etc.) with an antigen, collecting immune serum from the animal, and separating the polyclonal antibodies from the immune serum, in accordance with known procedures.

Monoclonal antibodies used to carry out the present invention may be produced in a hybridoma cell line according to known techniques.

"Antibody fragments" included within the scope of the present invention include, for example, Fab, F(ab')$_2$, and Fv fragments, and the corresponding fragments obtained from antibodies other than IgG. Such fragments can be produced by known techniques.

The term "extracellular marker" as used herein refers to markers expressed on the surface of cells.

The term "tissue-specific extracellular marker" as used herein refers to a marker that is expressed on the surface of a cell belonging to a certain type of tissue, e.g. tissue of epithelial or mesenchymal origin.

The term "cancer-specific extracellular marker" refers to a marker expressed on the surface of cancer cells, while the term "cancer-type specific extracellular marker" refers to a marker expressed on the surface of a certain type of cancer cell, i.e. on the surface of a colon cancer cell, a breast cancer cell, etc. The term "cancer-subtype specific extracellular marker" refers to a marker expressed on the surface of cancer cells, where the marker is indicative for a specific characteristic of the cancer cells, such as, e.g. aggressiveness, metastatic properties, invasiveness, capability of immunosuppression, chemotherapy sensitivity, and presence of tumour antigens relevant for Immunotherapy.

By the term "sentinel lymph node" is intended to mean the first lymph node(s) to receive lymphatic drainage from a tumour. The term "metinel lymph node" refers to the first lymph node(s) to receive lymphatic drainage from a metastasis.

By the term "true positives" is intended to mean a test sample correctly tested positive.

By the term "false positives" is Intended to mean a test sample falsely tested positive.

By the term "true negatives" is intended to mean a test sample correctly tested negative.

By the term "false negatives" is intended to mean a test sample falsely tested negative.

By the term "sensitivity" is intended to mean a measure that is calculated using the following formula:

$$Sensitivity = \frac{True\ positives}{True\ positives + False\ negatives}$$

By the term "specificity" is intended to mean a measure that is calculated using the following formula:

$$Specificity = \frac{True\ negatives}{True\ negatives + False\ positives}$$

By the term "negative predictive value (NPV)" is intended to mean a measure that is calculated using the following formula:

$$NPV = \frac{True\ negatives}{True\ negatives + False\ negatives}$$

By the term "reproducibility" is intended to mean the ability of a test to be independently reproduced with the same test results, such as, e.g., an inter- and intra-assay variability less than 10%.

By the term "labelling of agents" is intended to mean conjugation of an agent to a molecule detectable by an instrument, such as, e.g., enzymes, fluorescent molecules, radioactive molecules, biotin, oligonucleotides, and/or polysaccharides.

By the term "cut-off value" is intended to mean a value which is considered to be a value that gives a proper distinction between positive and negative patient cases (health status).

The panel of agents may be used for detecting cancer cells present in a human or animal body, such as, e.g. in the blood or in tissues. However, in a specific embodiment, the application relates to use of a panel of agents binding to extracellular markers, wherein at least one of the agents is cancer specific, for detecting disseminated cancer cells in tissues.

Even though the panel of agents may be used for detecting cancer cells anywhere in the human or animal body, the use of the panel of agents will in the following be exemplified and described in further details for detecting cancer cells in the lymphatic system, without limiting the invention in any way to the specifically described.

As mentioned above, the evaluation of presence or absence of tumour cells in the sentinel or metinel lymph nodes are very important for staging of the cancer and also has impact on the treatment strategy selected. If status of the sentinel or metinel node(s) can be rapidly determined it can also provide valuable information for decision-making during surgery. Accordingly, the invention relates to a method for detecting a cancer cell or cancer cell element in a biological sample, the method comprising adding to said biological sample a panel of agents, which bind to three or more different extracellular markers, wherein at least one of the agents is cancer specific. Detection of cancer cells or cell elements is executed by a spectrometric method, namely flow cytometry.

As mentioned above, the known techniques available for detecting cancer cells in sentinel or metinel lymph nodes have many drawbacks.

*Advantage: Higher sensitivity*

In the histopathological and immunohistochemical methods used today for examination of lymph nodes, the lymph nodes are cut into thin frozen sections and stained. However, as only a small fraction of the lymph node is examined, micro-metastasis outside the sections chosen will inevitably not be detected. Also, even if tumour cells are present within the sections manual screening by histopathological and immunohistochemical methods frequently misses small metastases (s 0.10 mm) and individual cancer cells (Weaver et al.)

In the present invention, a single-cell suspension of cells from the sentinel or metinel node(s) Is prepared after the sentinel or metinel lymph node(s) are removed. Samples of the single cell suspension are incubated with a panel of agents targeting extracellular markers and a high number of cells, such as, e.g. 100,000 cells or more are then analyzed by a method capable of quantifying the number and proportion of individual cells expressing specific extracellular markers. Accordingly, by using a suspension of cells from the one or more sentinel or metinel lymph node(s) a randomized population of cells representative for the one or more whole lymph node(s) is provided. Furthermore, analysis of a relatively high number of cells provides statistically secure evidence of absence of presence of tumour cells.

Accordingly, one of the advantages the present invention provides over the known techniques is a very high sensitivity. i.e. by the use of a panel of agents as described herein, the cancer cells may be detected with a sensitivity of 90% or more, such as, e.g., 91% or more, 92% or more, 93% or more, 94% or more, 95% or more, 96% or more, 97% or more, 98% or more, 99% or more or 100%, meaning that 90% or more, such as, e.g., 91% or more, 92% or more, 93% or more, 94% or more, 95% or more, 96% or more, 97% or more, 98% or more, or 99% or more, or 100% of sentinel or metinel nodes containing cancer cells will be correctly diagnosed as positive.

The advantage of higher sensitivity using the present invention is increasingly significant with decreasing size of metastases. As the present invention analyses a high number of individual cells, the probability of detecting a metastasis is only dependent upon the total number of tumour cells present in the lymph node. For conventional methods, the probability of detection has been shown to be dependent also upon the size of the metastasis. As an example, the sensitivity of detection by anti-cytokeratln immunohistochemistry has been found to be only 53% for breast cancer sentinel nodes initially classified as negative by histopathotogical screening. The risk of missing a metastasis was found to increase with decreasing size. The probability of missing a metastasis $\leq 0.10$ mm was found to be 61%. For metastases $\leq 0.05$ mm and $\leq 0.02$ the probability of missing the presence of tumour cells was 69% and 75%, respectively (Weaver et al.).

Overall sensitivity of histopathology with hematoxylin and eosin staining is approximately 50%. For screening with histopathology followed by anti-cytokeratin immunohistochemistry the overall sensitivity is approximately 80%. I.e. the present method provides a much higher sensitivity than the currently used methods of identifying tumour cells in a lymph node sample.

*Advantage: Higher specificity*

Another advantage of the present invention is that by analysing several markers at a time, more detailed information about each sample is obtained, thus reducing the risk of false-positive results by taking into account individual variations in cell expression. The method also does not rely upon detection of cytokeratin-20 which as described earlier is not a cancer-specific marker, which further reduces risk of a false-positive diagnosis.

i.e. by using a panel of agents as described herein, the cancer cells may be detected with a specificity of 95% or more, such as, e.g., 96% or more, 97% or more, 98% or more, or 99% or more, or 100% meaning that 95% or more, such as, e.g., 96% or more, 97% or more, 98% or more, or 99% or more, or 100% of the sentinel or metinel lymph nodes being free of cancer cells will be correctly diagnosed as negative.

Another way to express the improved sensitivity and specificity of the present invention is by the negative predictive value, i.e. the probability of a sentinel or metinel node diagnosed as free of cancer cells to be correctly diagnosed, This value reflects the combined high sensitivity and specificity. While other known techniques currently used today are prioritizing specificity to sensitivity, the present invention allows for maintaining high levels of both sensitivity and specificity.

Accordingly, in the present invention it is possible to combine a high sensitivity and specificity, i.e. cancer cells may be detected with a very high negative predictive value.

Accordingly, the present invention relates to a use, wherein the cancer cells may be detected with a negative predictive value of 90% or more, such as, e.g., 91% or more, 92% or more, 93% or more, 94% or more, 95% or more, 96% or more, 97% or more, 98% or more, or 99% or more, or 100% meaning that if a sample is identified as being negative for cancer cells by the method described herein, the probability of that sample actually being negative is 90% or more, such as, e.g., 91 % or more, 92% or more, 93% or more, 94% or more, 95% or more, 96% or more, 97% or more, 98% or more, or 99% or more, or even 100%.

A high negative predicted value is favourable in a method as described herein as the risk of the patient having removed any healthy tissue during surgery is greatly diminished.

*Advantage: Higher reproducibility*

Another advantage of the present invention is that it is easy to perform with a high reproducibility. Thus, the inter- and intra-assay variability is less than 10%, such as, e.g., less than 9%, less than 8%, less than 7%, less than 6%, less than 5%, less than 4%, less than 3%, less than 2%, or less than 1%. Accordingly, the procedure may be standardised and executed by a broad range of processionals, such as, e.g., biomedical scientists and clinical laboratory scientists. In comparison, the currently used histopathological methods requires the presence of a skilled pathologist which also leads to a much more subjective evaluation of the samples as there may be up to 30-35% variability between the observations of two different pathologists.

*Advantage: Faster*

Another advantage of the present invention is that the analysis of presence or absence of cancer cells in a human or animal sample may be performed in very short time. This is especially relevant if the method is used for intraoperative diagnosis of lymph node metastasis and micrometastasis, allowing the diagnosis to be provided during e.g. surgery of the primary tumour.

Accordingly, in the present invention the cancer cells may be detected within 25 minutes or less, such as, e.g. within 20 minutes or less, within 15 minutes or less, within 10 minutes or less.

By comparison, the known technique of immunohistochemical staining commonly requires several hours to complete.

Staining of cells by using the intracellular marker cytokeratin-20 suffers from the drawback that the cells have to be permeabilised with saponin to allow the agents to enter the cells, thereby adding time to the procedure. The average time for performing an analysis using cytokeratin-20 is about 45 minutes. Accordingly, by using agents binding to extracellular markers the time of the analysis is reduced significantly.

Conventional histopathological analysis of frozen tissue sections may enable a rapid diagnosis within maybe 20 minutes, but the sensitivity of such method for detecting e.g. micrometastasis is much lower than the sensitivity of the panel described herein as only a small fraction of the lymph node is examined, and the evaluation of the samples are subjective as described above. Experiments have also been performed using RT-PCR for diagnosis of lymph node metastasis, leading to a high sensitivity, however the amount of time commonly required to conduct such assays makes them completely unsuitable for intraoperative decision making. In comparison to that, the use of a panel according to the present invention provides a very high sensitivity and specificity combined with a short analysis time.

*Use of panels and selection of agents*

Detection of cancer cells by their phenotypic properties can follow two different "strategies". The first strategy is to look for markers that changes level of expression when the cell becomes cancerous. With support from data showing that a certain cancer type frequently expresses a certain marker while the normal cell originating from the same tissue does not, one is able to make the assumption that the cell is cancerous. In this case, the marker could be called "cancer specific" or "cancer-type specific".

The second strategy is to look for ectopic cells, i.e. to look for markers that should normally not be expressed in the tissue where analysed cells were collected. With support from data that a certain marker normally is expressed in one type of tissue and not expressed in the tissue where the cell was collected, one again is able to make the assumption that the cell is cancerous. In this case, you are looking for "tissue specific markers".

However, detection of cancer cells from their phenotype is not a straightforward task. Cancer cells originate from normal, "healthy", cells and naturally many of the surface markers are shared between the normal cell and the cancerous cell. In addition, cancer cells display an immature phenotype expressing developmental proteins, proteins that at early differentiation stages are shared between many cell lineage committed stem cells. The difference often lies in the level of expression of some of the markers.

Also, since the event of a normal cell becoming a cancerous cell is caused by genetic dysfunctions, the expression profile of a cancer cell is unique for each Individual and may even vary between cancer cells of the same origin.

In conclusion, the same marker can in some cases provide evidence that a cell expressing that marker is a cancer cell, and in other cases not To appoint a certain marker a "cancer marker" must thus be made with additional information, such as, what type of cancer should be detected, to what frequency a certain marker is expressed by a certain cancer type, and in what tissue the cell expressing the marker was found.

Detection of cancer cells should thus ideally take into account individual variations. One certain marker may be expressed by many cells of a certain cancer type, but not all cells. Cancerous cells originating from a certain tissue may lose expression of a certain marker normally expressed by that tissue.

In contrast to relying upon only one marker for detection of cancer cells, use of panels of agents binding to several markers reduces risk of both false-positive and false-negative results. Use of the present invention allows for detection of several cancer specific markers, cancer-type specific markers and tissue specific markers simultaneously. Failure of a cancer cell to express one certain marker may then be compensated by detection of another marker.

In the application of the present invention for detection of cancer cells in sentinel nodes, metinel nodes, or other lymph nodes, it is also possible to use the primary tumour as a positive control to verify the test. This further reduces the risk of a false test result.

*Known primary tumour: selection of agents*

One setup wherein the panel described herein is very useful is for detection of spread of cancer, i.e. the presence of metastasis including micrometastasis in the lymphoid system, such as in the sentinel and/or metinel lymph nodes.

The panel for use in such a setup should preferably contain one or more agents binding to general tissue-specific extracellular markers, such as, e.g., epithelial and/or mesenchymal extracellular markers. Almost all cancer cells will express at least one of these markers as they are derived from either epithelial or mesenchymal cells. In contrary, most cells present in a healthy lymph node do not normally express such markers. Accordingly, by including at least one agent binding to a general epithelial and/or mesenchymal marker, it is ensured that any cell not normally present in the lymph node is detected. However, as some non-cancerous cells having tissue-specific extracellular markers may also be present in lymph nodes (for example naevus cells containing mesenchymal extracellular markers) it is important to include at least one cancer-specific agent in the panel.

Accordingly, the panel must contain one or more agents binding to cancer-type specific extracellular markers, wherein the cancer-type specific marker is carbohydrate antigen 19-9 (CA19-9). In principle, any such agent can be used that is already identified or at present unknown.

Markers that can be used include specific extracellular markers, general epithelial markers, epithelial specific antigen, epidermal growth factor, mesenchymal extracellular markers, cancer-type specific markers, and cancer-subtype specific markers as well as markers for "solid tumours and other types of tumours", colon cancer cells, urinary bladder cancer cells, malignant melanoma cells, breast cancer cells, prostate cancer cells, ovarian cancer cells, lung cancer cells. Specific examples of markers are BC-10, carbohydrate antigen 125 (CA125), carbohydrate antigen 15-3 (CA15-3), carbohydrate antigen 19-9 (CA19-9), carbohydrate antigen 242 (CA242), carcino embryonic antigen (CEA), Ep-CAM, G250, MDR1, MDR2, MSH-R, NY-ESO-1, PRL-R, PSMA, RCAS1, STN, TA-90, and Beta 2-microglobulin (B2M), wherein Ep-CAM and CA19-9 are obligatory.

Agents that can be used include agents binding to tissue-specific extracellular markers, general epithelial markers, mesenchymal extracellular markers, cancer-type specific, and/or cancer-subtype specific extracellular markers and agents binding to cancer-type specific extracellular markers selected from markers for breast cancer, colon cancer, esophagus cancer, kidney cancer, liver cancer, lung cancer, malignant melanoma, ovary cancer, pancreas cancer, prostate cancer, rectum cancer, stomach cancer, thyroid cancer, or any combination thereof. Furthermore, the possible agents include monoclonal antibodies or fragments thereof binding one or more of the markers characteristic for colon cancer, malignant melanoma cells, breast cancer cells, prostate cancer cells, ovarian cancer cells, lung cancer cells. Specific examples of agents are monoclonal antibodies to carcino embryonic antigen (CEA), carbohydrate antigen 15-3 (CA15-3), carbohydrate antigen 125 (CA125), carbohydrate antigen 19-9 (CA19-9), carbohydrate antigen 242 (CA242), Ep-CAM, G250, RCAS1, STN, TA-90, BC-10, NY-ESO-1, MSH-R, PRL-R, and PSMA. One or more of the agents binds to CA19-9 and one or more of the agents binds to Ep-CAM.

As mentioned above, agents binding to markers characteristic for a specific subtype of cancer cells (cancer-subtype specific extracellular markers) may also be included in the panel. Examples of such markers may be markers indicating the following characteristics of the tumour/tumour cells, namely e.g. aggressiveness, metastatic properties, invasiveness, capability of immunosuppression, chemotherapy sensitivity, and presence of tumour antigens relevant for immunotherapy. By analysing for the presence of such markers, the treatment of the cancer may be tailored based on the specific tumour subtype. It is for example known that there is a series of membrane proteins called Multi Drug Resistance (MDR) proteins which may be heavily expressed in cancer cells and are able to pump anticarcinogenic agents out of the cancer cell, leaving the cancer cells insensitive or less sensitive to anticarcinogenic substances. By including one or more markers binding to MDR it is possible to determine whether the cancer cells will be sensitive towards treatment with chemotherapeutics and if so, administer chemotherapeutics as part of the same treatment regimen. However, if cancer cells prove to be insensitive to chemotherapeutics, other treatment regimens can be set up immediately.

*Unknown primary tumour: selection of agents*

Another use of a panel as described herein is for identifying the origin of a cancer metastasis. I.e. a situation where one or more metastases are identified in a patient, however, the location of the primary tumour having metastasized is not known.

Theoretically primary tumours originate from the cells present in the organ or tissue in which the tumour develops. A metastasis of a primary tumour is defined as a cancer resulting from the spread of a primary tumour. The metastasis process depends on the cancer cells acquiring two separate abilities - increased motility and invasiveness. Cells that metastasize are basically of the same kind as those in the original tumour. If a cancer arises in e.g. the colon and metastasizes to e.g. the liver, the cancer cells in the liver are colon cancer cells. However, the cells have acquired increased motility and the ability to invade another organ.

By using a panel according to the invention containing one or more agents binding to cancer-type specific and/or cancer sub-type specific extracellular markers it may be possible to determine the origin of the metastasis, i.e. to determine in which tissue the primary tumour have developed.

The panel may further comprise one or more agents binding to tissue-specific extracellular markers, such as, e.g., general epithelial and/or mesenchymal extracellular markers.

Metastasis may be found anywhere in the human or animal body, however, the most common sites to find a metastasis is in the lymph nodes, liver, lungs, skeleton, brain, ovaries, adrenal glands, and abdominal cavity.

Accordingly, the panel must contain one or more agents binding to cancer-type specific extracellular markers.

In principle, any such agent or marker can be used that is already identified or at present unknown. In the following examples are given without limiting the invention thereto.

Markers that can be used include specific extracellular markers, general epithelial markers, epithelial specific antigen, epidermal growth factor, mesenchymal extracellular markers, and cancer-type specific markers as well as markers for colon cancer cells, urinary bladder cancer cells, malignant melanoma cells, breast cancer cells, prostate cancer cells, ovarian cancer cells, lung cancer cells. Specific examples of markers are BC-10, carbohydrate antigen 125 (CA125), carbohydrate antigen 15-3 (CA15-3), carbohydrate antigen 19-9 (CA19-9), carbohydrate antigen 242 (CA242), carcino embryonic antigen (CEA), Ep-CAM, G250, MDR1, MDR2, MSH-R, NY-ESO-1, PRL-R, PSMA, RCAS1, STN, TA-90, and Beta 2-microglobulin (B2M). At least one of the agents binds to CA19-9 and at least one of the agents binds to EpCAM.

Agents that can be used include agents binding to tissue-specific extracellular markers, general epithelial markers, mesenchymal extracellular markers, cancer-type specific, and/or cancer-subtype specific extracellular markers as well as agents binding to cancer-type specific extracellular markers selected from markers for breast cancer, colon cancer, esophagus cancer, kidney cancer, liver cancer, lung cancer, malignant melanoma, ovary cancer, pancreas cancer, prostate cancer, rectum cancer, stomach cancer, thyroid cancer, or any combination thereof. Furthermore, the possible agents include monoclonal antibodies or fragments thereof binding one or more of the markers characteristic for colon cancer, malignant melanoma cells, breast cancer cells, prostate cancer cells, ovarian cancer cells, and lung cancer cells. Specific examples of agents are Monoclonal antibodies to carcino embryonic antigen (CEA), carbohydrate antigen 15-3 (CA15-3), carbohydrate antigen 125 (CA125), carbohydrate antigen 19-9 (CA19-9), carbohydrate antigen 242 (CA242), Ep-CAM, G250, RCAS1, STN, TA-90, BC-10, NY-ESO-1, MSH-R, PRL-R, and PSMA. The panel comprises agents binding to CA19-9 and Ep-CAM.

*Labelling of agents*

As an important step in use of the panel described herein is identification of the number of cells carrying specific markers, it may be advantageous to label the agents with one or more detectable labels. Such labels may be from enzymes, fluorescent molecules, radioactive labels, biotin, and/or oligonucleotides.

In a preferred embodiment of the invention, the agents are labelled with fluorescent molecules such as, e.g. nucleic specific dyes, phycobiliproteins, cyanine derivatives, fluorescein derivatives, and rhodamine derivatives. The different agents used in the panel are preferably labelled with different labels, so they can be distinguished from one another. When fluorescent molecules are used for labelling, the method may preferably be performed by a fluorescence measuring instrument, such as, e.g. a flow cytometer.

*Typical procedure*

Sentinel lymph nodes from a patient with cancer indication are obtained by surgery. Briefly, the sentinel node is identified during surgery by peri-tumoral injections of a tracer substance, e.g. patent blue dye, which follows the lymphatic drainage and colours the first-draining lymph nodes blue.

Single cell suspensions from the whole sentinel lymph node/s to be investigated are obtained by gentle pressure of the lymph nodes using a loose fit glass homogenizer in 5 ml PBS supplemented with 2% Fetal calf serum and 0.05% Sodium Azide (staining buffer) or in cell culture medium such as X-Vivo 15 or Aim 5.

Samples of $1x10^8$ cells are washed in 2 ml staining buffer..

Cell samples are then labelled in 100 $\mu$l staining buffer with a panel of fuorophore conjugated antibodies selected from tissue-specific, cancer-type specific and cancer-subtype specific extracellular markers and incubated at room temperature 15-30 minutes. The sufficient amount of antibody used is determined by titration of each antibody using a positive control. Typically this amount is in the range of 0.1-0.5 $\mu$g. To remove abundant antibody, samples are washed with 2 ml staining buffer and resuspended in 300 $\mu$l staining buffer before flow cytometric analysis.

The analysis is performed using instruments such as FACSAria, FACSCanto (Becton Dickinson) or Cytomics FC 500 (Beckman Coulter). Row cytometric analysis is performed collecting 100.000 events from each cell sample.

*Kits*

In order to facilitate use of the present invention, specialised kits can be developed for numerous applications of the present invention. Such kits could include all necessary agents, labels and fluids and instructions necessary to perform a certain test. The kit could also include software to facilitate interpretation of the test results, with e.g. predefined cut-off rates between a positive and negative test result

In a preferred embodiment of the present invention, a kit would include a panel of fluorescence labelled monoclonal antibodies, where the panel would be suitable for a certain type of test. Furthermore the kit would include fluids necessary to perform the test using flow cytometry and software capable of reliably interpreting the data generated from the analysis.

*Determining of cut-off values, prevalence and use of database*

Use of a software for interpretation of data generated by each test is dependant upon reliable statistical methods and background data for finding the optimal cut-off value for each test, as sensitivity and specificity are variable as the cut-off value between a positive and negative test result varies.

Background data may be obtained by performing tests on a large number samples with known status or known prevalence in the relevant population of the abnormality searched for. Data generated by each test, e.g. the proportion of events tested positive for each marker, will be collected along with information for categorisation of the population to which the test subject belongs, e.g. sex, age, disease, stage of disease, etc. The data generated may be used for finding optimal cut-off values.

Cut-off values are needed as also true negative samples will show varying degrees of "positivity" due to biological (varying degrees of expression of markers also in normal state) and technical (background noise) reasons.

In the case where the background data has been generated using samples with known status (positive or negative) finding of optimal cut-off values is relatively simple and can be achieved by analysing how sensitivity and specificity of the test is correlated to the cut-off value.

One approach to choice of cut-off Is to plot sensitivity versus (1 - specificity) for each possible cut-off value, i.e. several different cut-off values are first arbitrarily or methodically chosen. The curve obtained is known as the "Receiver Operating Characteristic" curve, ROC curve. Accuracy of the test is measured by the area under the ROC curve. An area of 1 represents a "perfect test", and an area of 0.5 represents a "worthless test".

If the tolerance of a false negative result is equal to that of a false positive result, the cut-off value which maximises the area under the ROC curve should be chosen. However, in many cases the clinical use of a test must be taken into account when deciding the cut-off value. For a screening test, e.g., it is important to minimise the number of false negative results and a moderate number of false positives Is acceptable, and the cut-off value will be set accordingly, i.e. the cut-off values may be chosen with respect to the clinical use/purpose.

ROC curves cannot be used alone to decide the cut-off values, as the method does not take the prevalence of the abnormality searched for into account. To optimise the ability of the test to predict the true disease state, background data on the prevalence of the abnormality to be detected is needed. The sensitivity and specificity of a test may vary with the prevalence of the condition searched for in the population to which the test subject belongs. Thus, it may be necessary to decide different cut-off values for different populations.

In the case where the true status of the sample is unknown, a different method for finding optimal cut-off values must be used. Reference intervals can be used for this purpose if the prevalence of the abnormality in different populations is known.

The reference interval is the estimated range of values that includes a certain percentage of the values among a relevant population. The most commonly used percentage is 95%.

Two reference intervals would have to be calculated for deciding of cut-off value, one interval for assumed negative samples and one interval for assumed positive samples. The categorisation of each sample would be made based on the prevalence of the abnormality in the population from where the sample has been collected. The proportional size of each sample group should conform to the prevalence of the abnormality in the population. Based on the test results for each node, each sample would be categorised into an assumed negative group and a assumed positive group. Reference intervals can be generated for each group, and the cut-off value may be set to minimise the overlap from one reference interval to the other. The cut-off value may also be set to minimise the risk of e.g. a false negative diagnosis.

Reference intervals can be calculated two basic approaches. If the data generated conforms to a distributional function such as, e.g., the Normal distribution, the reference intervals can be calculated using a parametric method meaning that the distribution conforms to a mathematically defined statistical distribution, e.g. the Normal distribution. Using parametric methods is generally preferred since confidence intervals of reference intervals are generally narrower unless the sample size is very large.

If the data generated does not conform to any mathematically defined statistical distribution, a non-parametric (distribution free) method would have to be used, wherein the estimated distributions is calculated using only the actual data generated by the analyses, in order to reach an acceptable cut-off value. In order to reduce the confidence interval a vary large sample size would be needed.

Prevalence of the abnormality to be detected may vary with e.g. age, sex, and disease/indication. Accordingly, access to large amounts of background data to determine reference intervals for each relevant population may further enhance the diagnostic performance when using the present invention for detection of tumour cells.

Comparing the data generated by an analysis with earlier data on true positive and true negative samples may optimise the cut-off rate between a positive and negative result, enabling even higher performance with respect to sensitivity, specificity and NPV.

In a preferred embodiment of the present Invention, interpretation of each test is performed by use of a software linked to a database with background data for each marker tested in relation to relevant populations. The software may also be capable of generating data for use in the same database, which would allow for continuous fine-tuning of cut-off values for each test and relevant population.

Accordingly, in separate aspects the invention relates to a database storing results obtained by the flow cytometry method claimed herein and software for calculating and continuously refining cut-off values when the data material increases. The database contains a plurality of data set, wherein each data set comprises data for a plurality of extracellular surface markers and corresponding type or types of cancer cells. Moreover, the invention relates to a method for identifying cancer cells as claimed herein further comprising a step of storing the obtained data set from the flow cytometry method (e.g. i) number of cells carrying an agent that binds to one or more extracellular markers, ii) type of cancer cells, iii) type of tissue and the like). The step of storing the data may further comprise a step of inputting data of known origin with corresponding data set.

Moreover, in a further aspect the present invention relates to a software program that when run on a computer is i) adapted to receive flow cytometry data set comprising data for extracellular markers, ii) adapted to compare the received data with data in a database, and iii) adapted to provide an output indicating a) the presence or absence of cancer cells, and optionally b) the type or types of cancer cells.

Example of how extracellular markers could be evaluated for comparison purposes in a database is given in example 4.

Figure legends

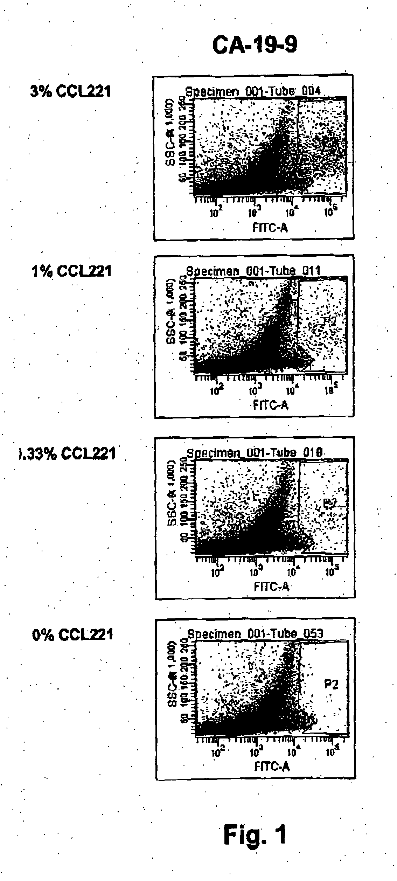

Figure 1 illustrates the identification by flow cytometry of colon cancer cells in a mixture of colon cancer cells and leukocytes using a single agent for identification. Various numbers of CCL221 colon cancer cells (from 0-3%) were introduced into peripheral blood leukocytes purified by Ficoll and stained using direct conjugated anti CA19-9 as cell surface detection antibody. The presence of tumour cells was detected by flow cytometry. SSC, side scatter, indicates granularity of cells.

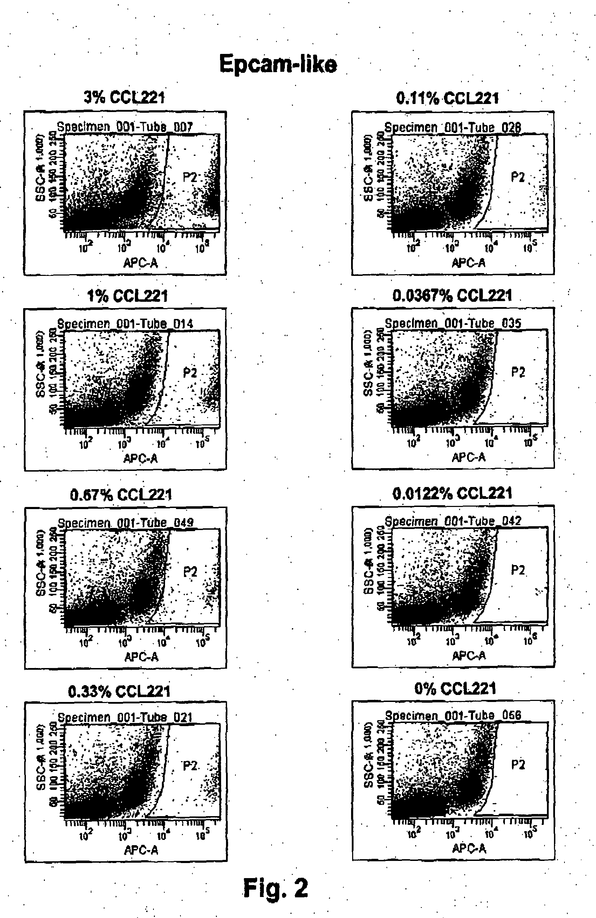

Figure 2 illustrates the identification by flow cytometry of colon cancer cells in a mixture of colon cancer cells and leukocytes using a single agent for identification. Various numbers of CCL221 colon cancer cells (from 0-3%) were introduced into peripheral blood leukocytes purified by Ficoll and stained using direct conjugated EpCAM-like antibody for cell surface detection. The presence of tumour cells was detected by flow cytometry. SSC, side scatter, indicates granularity of cells.

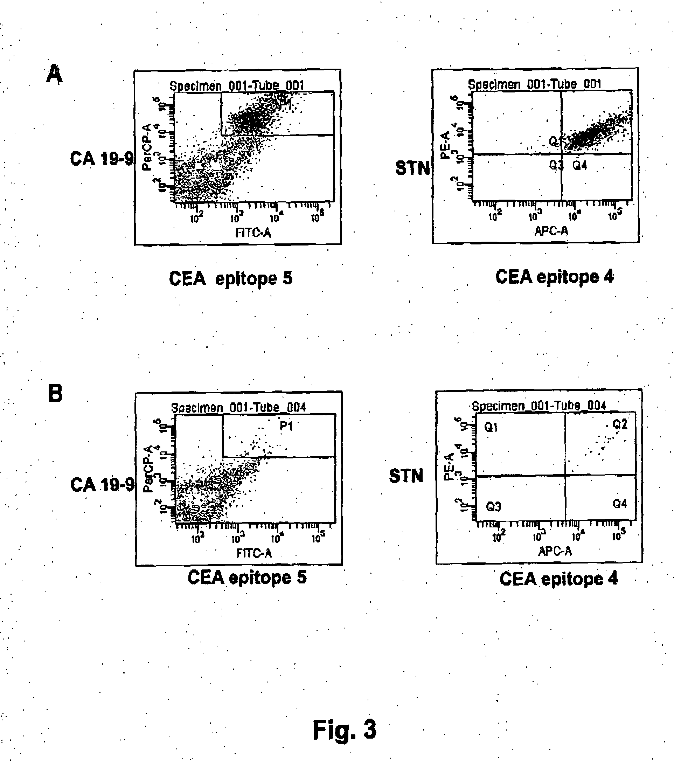

Figure 3 illustrates the identification by flow cytometry of colon cancer cells in a mixture of colon cancer cells and leukocytes using a panel of four agents for identification. CCL221 colon cancer cells 10% (A) or 1% (B) were introduced into peripheral blood leukocytes purified by Ficoll and stained using direct conjugated antibodies for simultaneous four colour detection of cell surface markers CA19-9, CEA epitope 5, and CEA epitope 4, and STN. The presence of tumour cells was detected by flow cytometry. Double positive cells recognized by CA19-9 and CEA epitope 5 (left panel) were electronically gated and evaluated by the second pair of antibodies STN and CEA epitope 4 (right panel).

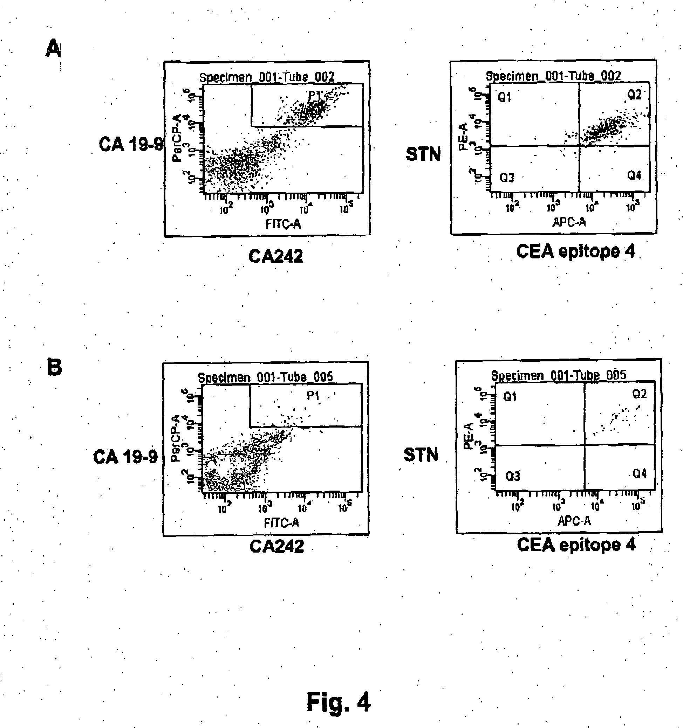

Figure 4 illustrates the identification by flow cytometry of colon cancer cells In a mixture of colon cancer cells and leukocytes using a panel of four agents for identification CCL221 colon cancer cells 10% (A) or 1% (B) were introduced into peripheral blood leukocytes purified by Ficoll and stained using direct conjugated antibodies for simultaneous four colour detection of cell surface markers CA19-9, CA242, and CEA epitope 4, and STN. The presence of tumour cells was detected by flow cytometry. Double positive cells recognized by CA19-9 and CA242 (left panel) were electronically gated and evaluated by the second pair of antibodies STN and CEA epitope 4 (right panel).

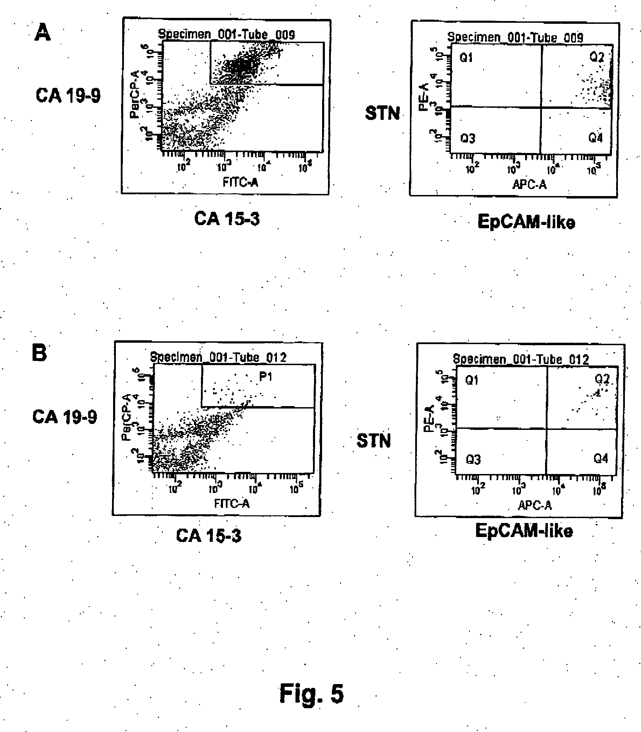

Figure 5 illustrates the identification by flow cytometry of colon cancer cells in a mixture of colon cancer cells and leukocytes using a panel of four agents for identification. CCL221 colon cancer cells 10% (A) or 1 % (B) were introduced into peripheral blood leukocytes purified by Ficoll and stained using direct conjugated antibodies for simultaneous four color detection of cell surface markers CA19-9, CA15-3, EpCAM-like, and STN. The presence of tumour cells was detected by flow cytometry. Double positive cells recognized by CA19-9 and CA15-3 (left panel) were electronically gated and evaluated by the second pair of antibodies STN and EpCAM-like (right panel).

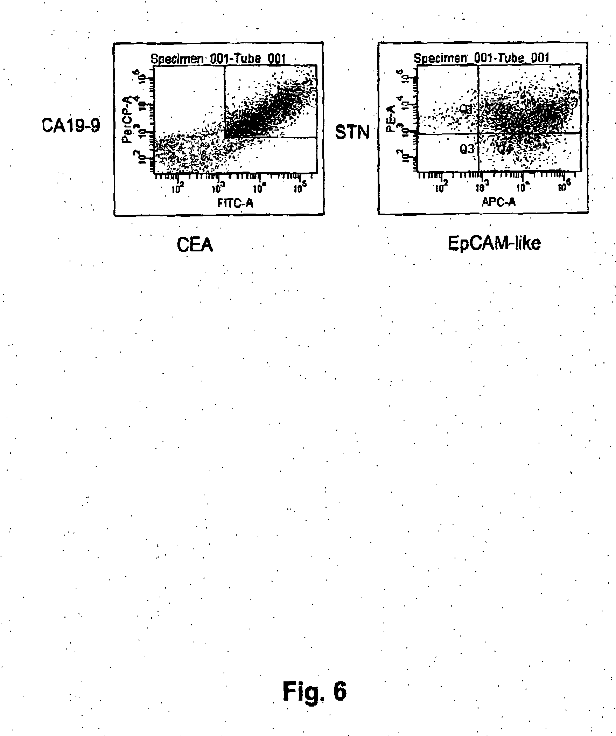

Figure 6 illustrates the identification by flow cytometry of colon cancer cells in a single cell suspension from a sentinel lymph node from a patient with colon cancer. The analysis was performed using a panel of antibodies recognizing four different colon markers CA19-9, CEA, STN, and EpCAM-like protein.

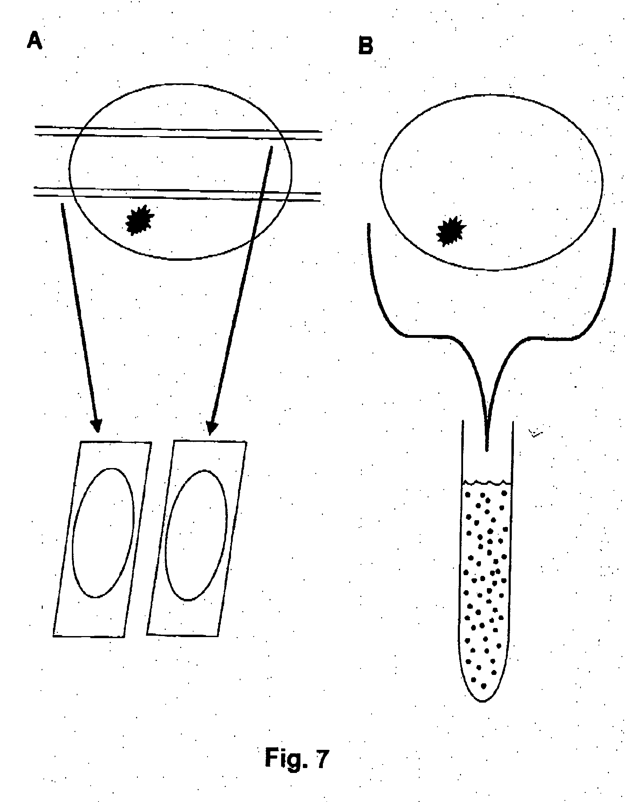

Figure 7 illustrates the decreased risk of a false-negative result when analysing a single cell suspension obtained from a lymph node instead of analysing thin sections of a lymph node by histochemistry. A illustrates a situation where a metastasis (indicated by a red cluster) ties outside the sections that are eventually stained and evaluated in microscope. The tumour cells In the lymph node will be absent from the sections, and the analysis will produce a false-negative result. B illustrates how a single cell suspension sample obtained from the whole lymph node represents the status of the whole lymph node. Analysis of a statistically relevant number of cells will produce a true-positive result

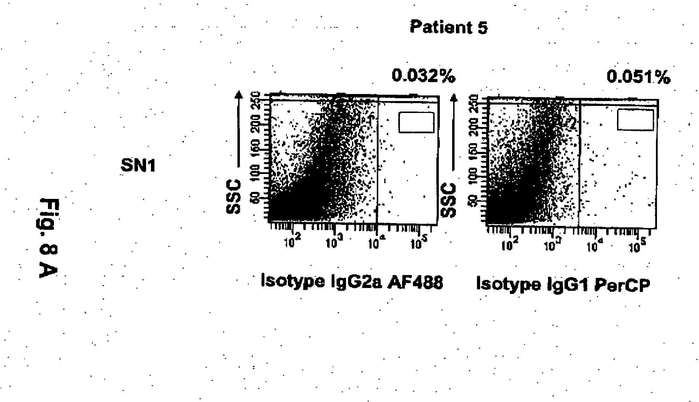

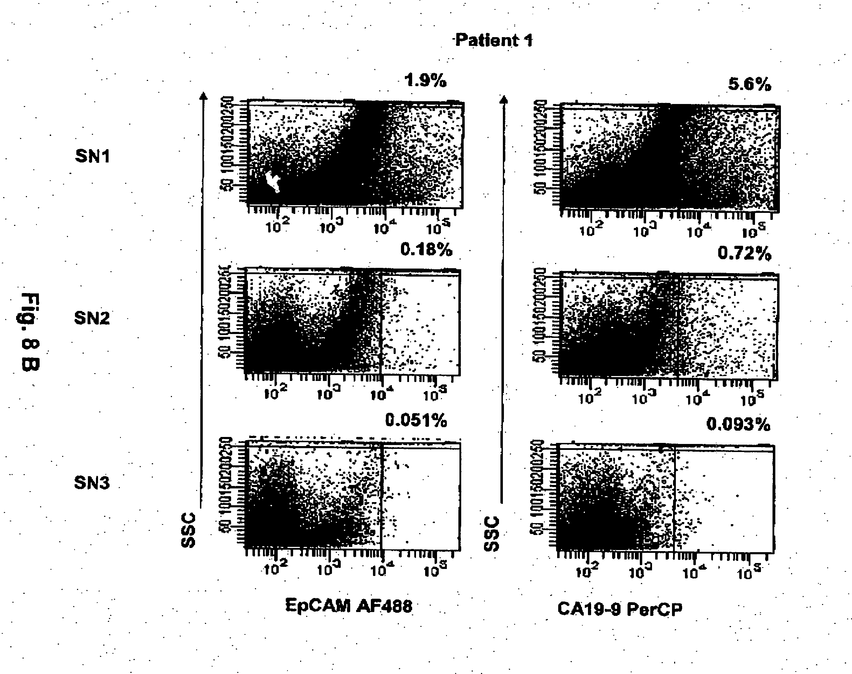

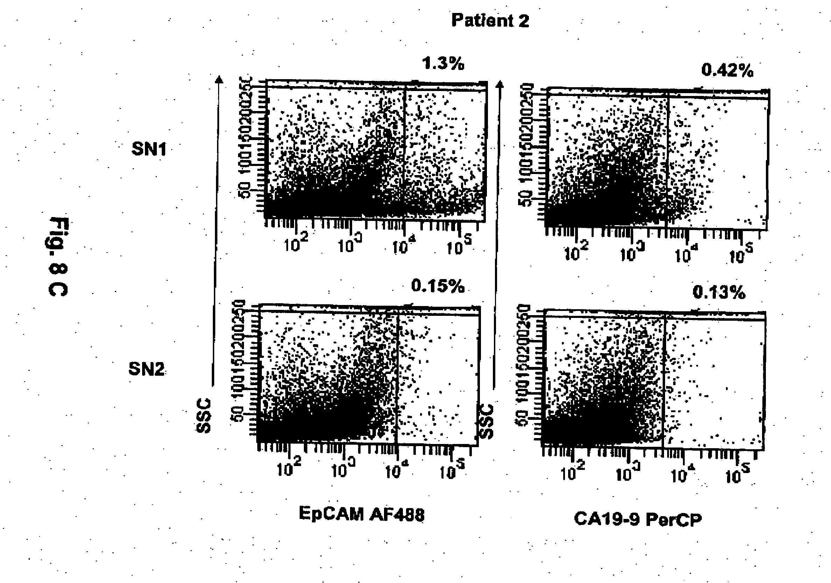

Figure 8 illustrates evaluation of reference intervals for markers EPCAM and CA 19-9 in a population of colon cancer patients, as described in Example 4. A illustrates results of isotype control stainings performed for each sentinel node stained. B illustrates results from stainings of sentinel nodes SN1, SN2 and SN3 from patient 1. Results indicate high and moderate levels of presence of both markers SN1 and SN2. respectively, whereas expression in SN3 is close to detection level. C illustrates results from stainings of sentinel nodes SN1 and SN2 from patient 2. Results indicate presence of both markers in SN1. In SN2 presence is close to detection level.

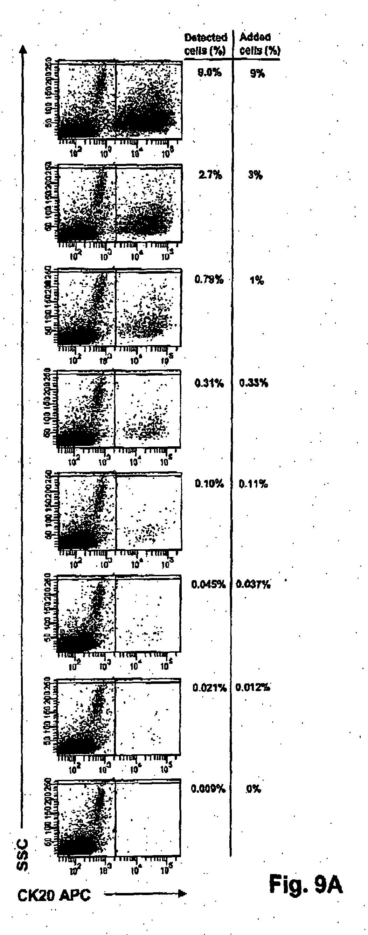

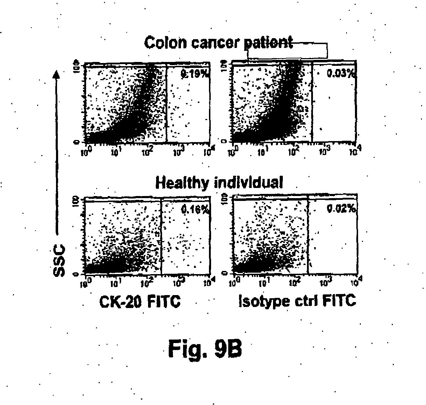

Fig. 9. Detection of tumour cells by flow cytometry after staining for CK20. A dilution series of PBMC spiked with DLD-1 colon cancer cells was stained intracallularly for the epithelial cell marker CK20 and analyzed using flow cytometry (1A). Findings of CK20 positive cells in PBMC from one colon cancer patient and one healthy individual are depicted (1B).

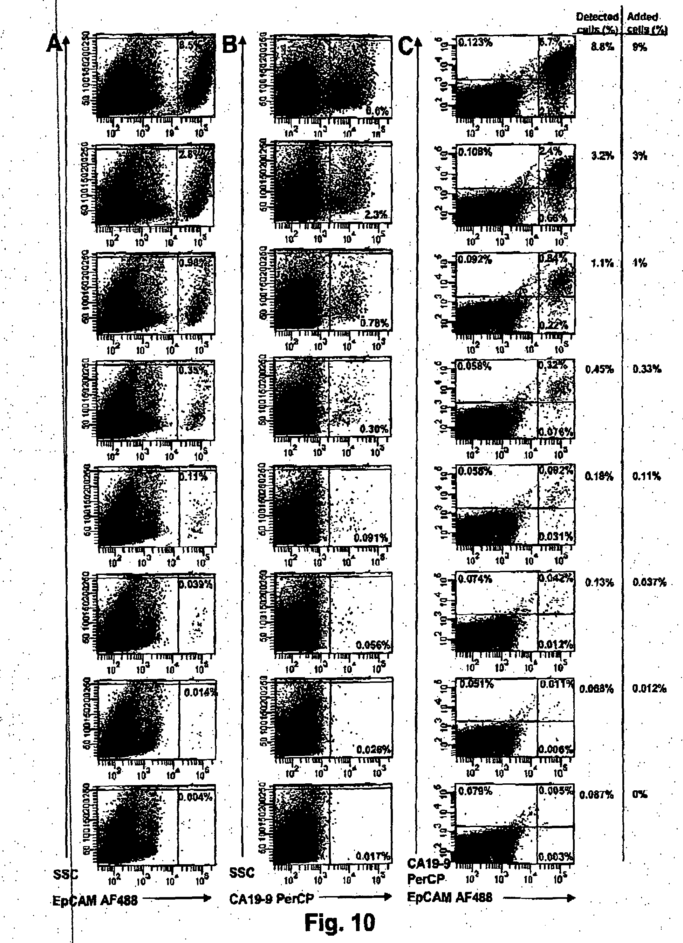

Fig. 10. Flow cytometric detection of tumour cells by staining for the tumour-associated cell surface markers EpCAM and CA19-9. The colon cancer cell line DLD-1 was diluted in PBMC in a dilution series range of 0.012-9% added tumour cells, PBMC alone being used as negative control. EpCAM expression was analyzed and plotted versus SSC properties; the number indicates the percentage of EpCAM$^+$ events (2A). Analysis of CA19-9 expression plotted versus SSC properties, the number indicating the percentage of events positive for CA19-9 (2B). Co-expression of the tumour markers was examined by plotting EpCAM versus CA19-9, the numbers in the plot indicating the percentage of events in the respective quadrants (2C). In the columns to the right of Fig. 2C the total number of detected cells, i.e. the sum of EpCAM$^+$CA19-9$^-$, EpCAM$^-$ CA19-9$^+$ and EpCAM$^+$CA19-9$^+$ cells, are presented as well as the percentage expected from the number of added tumour cells.

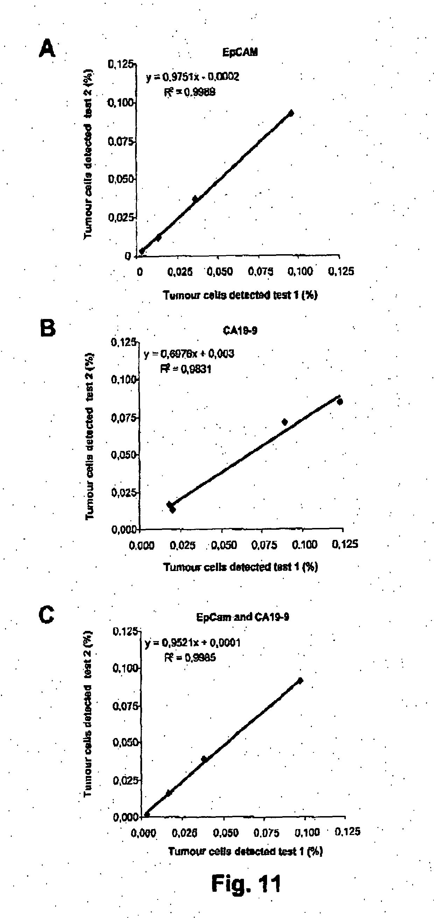

Fig. 11. Inter-assay analysis correlating two individual investigations. PBMC spiked with colon cancer cell line DLD-1 at the low range of added tumour cells (0, 0.012, 0.037, and 0.11 respectively), were analysed for inter-assay variability collecting $10^6$ events. Samples were stained with antibodies against EpCAM and CA19-9 and analyzed by flow cytometry. Regression analysis was performed comparing tests 1 and 2 for EpCAM expression (3A), CA19-9 expression (3B), and the EpCAM/CA19-9 expression (3C).

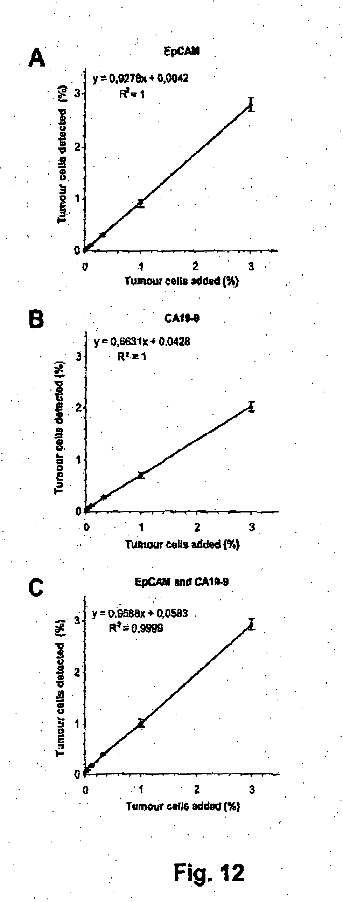

Fig. 12. Correlation between the detected and expected numbers of tumour cells. Samples of PBMCs spiked with colon cancer cell line DLD-1 (0.037, 0.11, 0.33, 1 and 3% respectively), and one sample with PBMCs alone, were split into 10 aliquots for assessment of intra-assay variability. Samples were stained with antibodies specific for EpCAM and CA19-9 and analyzed by flow cytometry. Regression analysis was performed for the percentage of EpCAM expression plotted versus SSC characteristics (4A) and for CA19-9 expression plotted versus SSC characteristics (4B). The total presence of single and double positive cells, i.e. the sum of EpCAM$^+$CA19-9$^-$, EpCAM$^-$CA19-9$^+$ and EpCAM$^+$CA19-9$^+$ cells, as evident in a dot plot of EpCAM versus CA19-9 was examined (4C). Data is presented as mean ($\pm$SD) percentage detected tumour cells on the y axis and the number of expected cells depicted on the x axis.

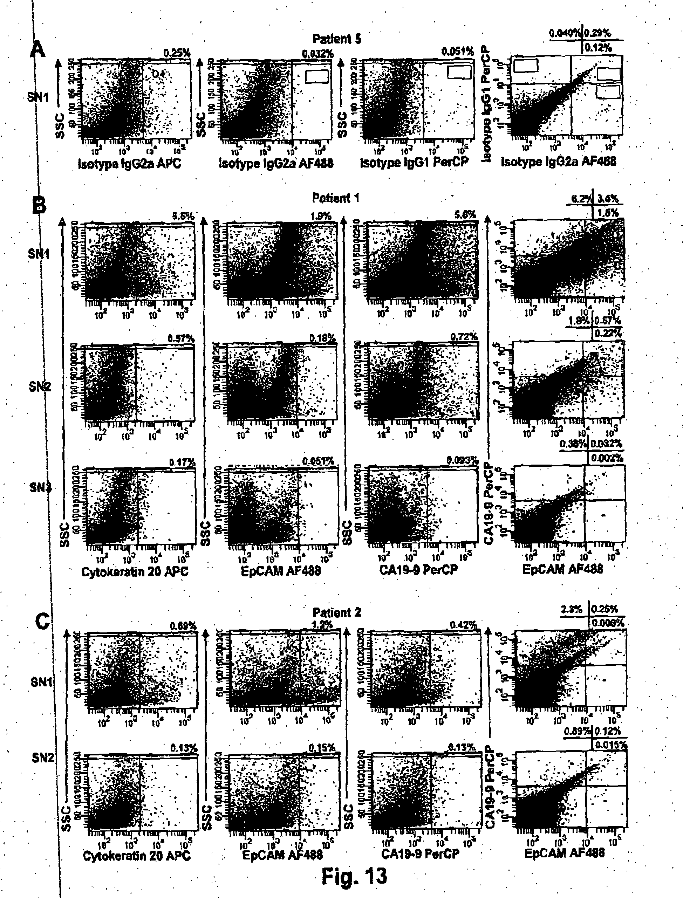

Fig. 13. Presence of colon cancer metastases in sentinel nodes. Fourteen sentinel nodes from seven patients diagnosed with colon cancer were intracellularly stained in parallel for CK20 or extracellularly for EpCAM and CA19-9, before flow cytometric analysis. For each sentinel node stainings with relevant isotype controls were performed, here exemplified with the staining of SN1 from patient 5 (5A). In patient one, three sentinel nodes were identified. Presence of metastatic cells was detected in SN1 and SN2 whereas SN3 only contained a low number of positive events dose to the level of detection and was considered free from metastases (5B). Flow cytometric analysis of the two sentinel nodes obtained from patient two indicates metastatic spread to SN1; in SN2 the number of positive events was dose to detection limit and SN2 was thus considered negative (5C).

**Examples**

**Example 1**

**Flow cytometric detection of colon cancer cells in a mixture of leukocytes and colon cancer cells**

*Results*

In order to evaluate single cell flow cytometry of cell surface stained tumour cells we added a known number of colon cancer cells from the colon cancer cell line CCL221 to peripheral blood leukocytes. Thus, we mimic the situation in a lymph node containing a small metastasis. First we used the tumour marker CA19-9, known to be expressed on the surface of colon cancer cells. Analysis of the sample was best performed using the side scatter (SSC) for size and granularity and FL-1 reflecting the FITC conjugate stained cells. Cells double positive with regards to an increased SSC and a high fluorescence was identified as exemplified in Figure 1. Even as low as 0.33% of added CCL221 cells was easily detected. However, a small number of unspecific staining was seen even when tumour cells were not present in the sample, Indicating a small fraction of false positive cells. In order to improve the assay the experiment was performed using an antibody recognizing an EpCAM-like cell surface molecule expressed on colon cells adding from 0.0122-3% tumour cells to the PBL. Again, the tumour population was easily detected as a cluster of cells expressing high amount of the EpCAM-like protein and an increased SSC (Figure 2). Even as few as 0.0122% cells were detected as a distinguished population of cells (Figure 2). There was no problem in false positive cells using the EpCAM-like protein as a targeted cell surface marker since no events was recorded as double positive when no tumour cells were added. Since it is well known that tumour cells may lose the expression of molecules with their loss of maturation, we wanted to combine a set of cell surface molecules to improve the diagnostic precision of the assay without the risk of missing tumour cells that have lost the expression of a certain cell surface molecule. We therefore tested combinations of four different cell surface markers. The combination of CA19-9 and two epitopes of CEA and STN detected the vast majority of cells being quadruple positive (Figure 3A, right panel). We noted that a small majority, less than 1% where triple positive (Figure 3A, right panel, Q1) suggesting that a few cells indeed have lost the expression of one of the CEA epitopes. When fewer cells (1%) were added to the PBLs the same result was obtained (Figure 3B). We then tested a different combination of four cell surface proteins expressed on colon cancer cells CA19-9, CA242, STN, and CEA epitope 4. This quadruple combination behaved in a similar fashion when recognizing the presence of CCL221 cell line in PBLs (Figure 4). Again, the majority of cells were positive for all the 4 markers CA19-9, CA242, STN, and CEA epitope 4 (Figure 4A, right panel, Q2), and the result was identical when fewer CCL221 cells were added (Figure 4B, right panel, Q2). A third combination of cell surface was tested CA19-9, CA15-3, EpCAM-like protein and STN was tested with similar results (Figure 5A and B). When the system was tested on a sentinel node derived from a colon cancer patient we could easily detect the presence of colon cancer cells by flow cytometry. Using markers CA19-9 and CEA identified 33% of the cells in the sentinel node as cells carrying colon cancer cell markers (Figure 6). Furthermore, electronically gated CA19-9[+] CEA[+] cells and analysing for STN and EpCAM-like expression revealed that 24% of the cells appeared quadruple positive expressing all the tested markers CA19-9[+] CEA[+] STN+ EpCAM-like+. However, 1.7% of the cells are triple positive for the combination CA19-9[+] CEA[+] STN[+] and 7.7% positive for the CEA19-9[+] CEA[+] EpCAM-like[+], demonstrating that colon cancer cells may lose the expression of some cell surface markers at various extent

**Table 1**

| Antibodies against surface markers on colon cancer cells | | |
|---|---|---|
| **Name** | **Fluorophore** | **Antigen** |
| B | Alexa647 | CEA |
| C | Alexa488 | CA242 |
| D | PerCP | CA19-9 |
| E | PE | STN |
| F | Alexa488 | CA15-3 |
| G | Alexa647 | EpCAM-like |

**Example 2**

**Flow cytometric detection of metastatic colon cancer cells in lymph nodes**

*Materials and Methods*

Preparation of cells

Periheral blood leukocytes where obtained by Ficoli paque. The colon cancer cell line CCL221 was grown as suggested by provider (ATCC). A sentinel lymph node from a patient being diagnosed with colon cancer was obtained by surgery. Single cell suspensions from the whole sentinel lymph node/s to be investigated were obtained by gentle pressure of the lymph nodes using a loose fit glass homogenizer.

Flow cytometry

Samples of $1 \times 10^6$ cells were washed in PBS supplemented with 2% Fetal calf serum and 0.05% Sodium Azide (FACS-buffer). Cell samples were then stained with a panel of fluorophore conjugated antibodies selected from tissue-specific, cancer-type specific and cancer-subtype specific extracellular markers (see examples of suitable markers in Table 1 above) and incubated at room temperature 15-30 minutes. To remove abundant antibody samples were washed with FACS-buffer before flow cytometric analysis. Results of the analysis were immediately available. The investigated lymph node was shown to contain a heavy burden of metastatic cells since a large proportion contained quadruple positive cells (Figure 6).

**Example 3**

**Flow cytometric detection of colon cancer cells in single cell samples and assay performance/robustness**

Single cell suspensions of sentinel node lymphocytes, tumour cells and PBMC were analysed from the investigated patients. To enable quantification of colon cancer cells samples of PBMC from healthy volunteers, spiked with the colon cancer cell fine DLD-1, were also investigated. A seven-step, 3-fold serial dilution series with a starting concentration of 9% tumour cells resulted in concentrations of 9%, 3%, 1%. 0.33%, 0.11%, 0.037% and 0.012%. A sample of PBMC alone was added to the series.

For Intracellular staining of the epithelial cell marker CK20 cells were first washed in PBS supplemented with 2% BGS and 0.05% (w/v) Sodium Azide (staining buffer) and thereafter treated with Cytofix/Cytoperm (Becton Dickinson) according to the manufacturer's instructions. Thereafter the cells were retained in staining buffer supplemented with 0.3% saponin (w/v) (Sigma). Primary staining was performed with mouse anti-human CK20 antibody (Dakocytomation) or IgG2a isotype control (Dakocytomation) for 30 min. Cells were washed and incubated for 30 min with a goat-anti-mouse antibody conjugated with allophycocyanine (APC) (Jackson Immunoresearch) or a donkey-anti-mouse antibody conjugated with fluorescein isothiocyanate (FITC) (Jackson Immunoresearch). Staining for cell surface markers was performed in staining buffer. Cells were washed and then incubated for 30 min with anti-epithelial cell adhesion marker (EpCAM) and anti-Carbohydrate Antigen 19-9 (CA19-9) (Fujirebio diagnostic), conjugated with Alexa fluor 488 (AF488) (Molecular probes Invitrogen) or Peridinin-chlorophyll-protein complex (PerCP) (Prozyme), respectively, according to the manufacturers' descriptions. Isotype controls used were IgG2a (clone G155-178) conjugated with AF488 and IgG1 (clone MOPC-21) conjugated with PerCP, (Becton Dickinson). Acquisition of 100,000 events (Table 2, Table 4) or 1,000,000 events (Table 3), respectively, from each sample was conducted using a FACSAria (Becton Dickinson) and the collected data were analysed using the FACSDiva software. Positive events were identified by plotting of respective fluorescence in log-scale against linear side-scatter (SSC) characteristics. The upper left and right qudrants were set to exclude dead cells and cell debris initially tested out with PI. The samples stained for both EpCAM and CA19-9 were also analysed using dot plots with log green fluorescence (AF488) versus log red fluorescence (PerCP)

Table 2. Intra-assay variability

| Expected cell numbers (%) | EpCAM+ | | | CA19-9+ | | | EpCAM+CA19-9+ EpCAM+CA19-9- EpCAM-CA19-9+ | | |
|---|---|---|---|---|---|---|---|---|---|
| | Mean (n=10) | SD | CV (%) | Mean (n=10) | SD | CV (%) | Mean (n=10) | SD | CV(%) |
| 3 | 2.8 | 0.129 | 4.6 | 2.0 | 0.080 | 4.0 | 2.9 | 0.107 | 3.7 |
| 1 | 0.93 | 0.066 | 7.2 | 0.71 | 0.057 | 8.0 | 1.0 | 0.072 | 7.2 |
| 0.33 | 0.32 | 0.018 | 5.8 | 0.27 | 0.019 | 6.9 | 0.39 | 0.015 | 4.0 |
| 0.11 | 0.11 | 0.011 | 10.7 | 0.11 | 0.015 | 12.9 | 0.17 | 0.016 | 9.4 |
| 0.037 | 0.041 | 0.006 | 15.5 | 0.067 | 0.007 | 10.4 | 0.093 | 0.009 | 9.8 |
| 0 | 0.002 | 0.002 | 90.0 | 0.036 | 0.006 | 16.1 | 0.047 | 0.005 | 9.8 |

Table 3. Intra- and inter- assay variability

| Expected cell numbers (%) | EpCAM+ 1st aquisition | | | EpCAM+ 2nd aquisiton | | |
|---|---|---|---|---|---|---|
| | Mean (n=10) | SD | CV (%) | Mean (n=10) | SD | CV (%) |
| 0.11 | 0.096 | 0.005 | 5.15 | 0.093 | 0.005 | 5.75 |
| 0.037 | 0.036 | 0.003 | 7.09 | 0.037 | 0.004 | 9.83 |
| 0.012 | 0.013 | 0.001 | 6.19 | 0.012 | 0.001 | 8.11 |
| 0 | 0.004 | 0.001 | 2.84 | 0.003 | 0.001 | 23.80 |

| Expected cell numbers (%) | CEA19-9+ 1st aquisition | | | CA19-9+ 2nd aquisition | | |
|---|---|---|---|---|---|---|
| | Mean (n=10) | SD | CV (%) | Mean (n=10) | SD | CV (%) |
| 0.11 | 0.124 | 0.015 | 12.23 | 0.085 | 0.006 | 6.53 |
| 0.037 | 0.089 | 0.016 | 17.87 | 0.072 | 0.011 | 14.82 |
| 0.012 | 0.018 | 0.002 | 8.70 | 0.017 | 0.001 | 4.29 |
| 0 | 0.020 | 0.002 | 11.07 | 0.013 | 0.001 | 10.07 |

| Expected cell numbers (%) | EpCAM+CA19-9+ EpCAM+CA19-9- EpCAM-CA19-9+ 1st aquisition | | | EpCAM+CA19-9+ EpCAM+CA19-9- EpCAM-CA19-9+ 2nd aquisition | | |
|---|---|---|---|---|---|---|
| | Mean (n=10) | SD | CV (%) | Mean (n=10) | SD | CV (%) |
| 0.11 | 0.097 | 0.007 | 6.91 | 0.092 | 0.005 | 5.64 |
| 0.037 | 0.038 | 0.004 | 9.85 | 0.039 | 0.005 | 11.89 |
| 0.012 | 0.016 | 0.002 | 13.54 | 0.016 | 0.002 | 11.13 |
| 0 | 0.004 | 0.001 | 31.34 | 0.002 | 0.001 | 41.70 |

Table 4. Investigation of sentinel nodes from patients with colon cancer

| Pat no. | Sentinel node no: | CK20+ (%) | EpCAM+ (%) | CA19-9+ (%) | EpCAM+CA19-9+ EpCAM+CA19-9- EpCAM-CA19-9+ (%) |
|---|---|---|---|---|---|
| 1 | SN1 | 5.6 | 1.9 | 5.6 | 11 |
| 1 | SN2 | 0.57 | 0.18 | 0.72 | 2.6 |

(continued)

| Pat no. | Sentinel node no: | CK20+ (%) | EpCAM+ (%) | CA19-9+ (%) | EpCAM+CA19-9+ EpCAM+CA19-9- EpCAM-CA19-9+ (%) |
|---|---|---|---|---|---|
| 1 | SN 3 | 0.17 | 0.051 | 0.093 | 0.41 |
| 2 | SN1 | 0.69 | 1.3 | 0.42 | 2.5 |
| 2 | SN2 | 0.13 | 0.15 | 0.13 | 1.0 |
| 3 | SN | 0.099 | 0.038 | 0.042 | 0.75 |
| 4 | SN1 | 0.17 | 0.088 | 0.024 | 0.80 |
| 4 | SN2 | 3.1 | 2.8 | 24.7 | 29 |
| 5 | SN1 | 1.9 | 0.79 | 1.7 | 3.9 |
| 5 | SN2 | 1.9 | 1.3 | 2.4 | 8.2 |
| 6 | SN1 | 0.20 | 0.26 | 0.078 | 1.1 |
| 6 | SN2 | 0.30 | 0.55 | 0.27 | 1.6 |
| 7 | SN 1 | 0.18 | 0.069 | 1.3 | 1.4 |
| 7 | SN2 | 0.18 | 0.064 | 0.65 | 0.76 |

*Assay performance, Robustness*

The intra-assay variability for flow cytometric analysis of tumour surface markers was tested by splitting a sample into ten aliquots before flow cytometric analyses. From the dilution series samples with 0.037%, 0.11%, 0.33%, 1% and 3% added DLD-1 tumour cells and samples of PBMC alone were examined. For EpCAM and CA19-9, when presented separately, the number of positive events was determined in dot plots of respective fluorescence in log-scale versus linear SSC properties. Analysis of the total number of detected cells was performed by plotting log green fluorescence versus log red fluorescence and the events recorded as either EpCAM+CA19-, EpCAM-CA19-9+ or Ep-CAM+CA19-9+ were accumulated. The resulting mean detection of colon cancer cells, standard deviation and coefficient of variation are listed in Table 2.

The mean values of tumour cells detected with EpCAM expression were close to those expected, indicating that almost all DLD-1 cells expressed EpCAM (Table 2). The CV values obtained from the samples with 0.11, 0.33,1 and 3% added DLD-1 colon cancer cells were in the range of 4.6-10.7%, indicating very low intra-assay variability. The mean number of detected cells for 0.037% added cells was 0.041 % (range 0.035-0.055%) and for PBMC with no addition of tumour cells 0.002 (range 0-0.004%) (Table 2). When CA19-9 expression was investigated the mean values of tumour cell recovery were lower than the expected numbers, again indicating that not all tumour cells express CA19-9 (Table 2). The average background staining in PBMCs was 0.036%, which interferes with detection of tumour cells at low concentrations, but for 0.33, 1 and 3% added cells 67,71 and 82%, respectively, appear to express CA19-9. For the range 0.037-3% added cells the average CV for CA19-9 detection was 8.4%. Indicating low intra-assay variability.

When the expression of EpCAM+CA19-9-, EpCAM-CA19-9+ and EpCAM+CA19-9+ was examined the resulting means are close to the expected numbers of cells (Table 2). In addition, $10^6$ cells/sample were collected to further validate the method at cell numbers close to the threshold (Table 3). The standard deviations for the measurements where low (range 0.001-0.016), demonstrating low intra-assay variability when testing 10 samples. The CVs for the detection of EpCAM+ and CA19-9+ cells for the whole range of added cells (0-0.11 %) were generally low, also indicating assay stability. However, when measuring cell numbers close to zero the coefficient of variation is mathematically sensitive to small changes in the standard deviation, limiting its usefulness for calculations regarding low number of cells. The comparison of test 1 and test 2 performance for tumour cell detection using EpCAM, CA19-9 and Ep-CAM/CA19-9 antibodies were in good agreement with regression coeffecients close to 1 (Fig. 11A-C).

The dynamic range from 0 to 3% cells was tested by performing regression analyses (Fig. 12A-C). The sample coefficient of determination was 1 for EPCAM (Fig. 12A), CA19-9+ (Fig, 12B) and for double positive cells. (Fig. 12C), demonstrating a stable and reliable performance within the investigated range. The correlation between observed numbers and expected number of tumour cells for EpCAM+ (Fig. 12A) and for the sum of EpCAM+CA19-9-, EpCAM-CA19-9+ and EpCAM+CA19-9+ cells (Fig. 12C) was close to what is expected from the theoretical number of added cells. CA19-9 appears to be expressed on approximately 73% of DLD-1 tumour cells.

**Example 4**

**Evaluation of markers in sentinel nodes from colon cancer patients**

*Materials and methods*

Sentinel lymph nodes from 7 patients were identified during surgery by peri-tumoral injections of a tracer substance. Single cell suspensions from each lymph node were prepared using a loose-fit glass homogeniser. Staining for extracellular markers was performed in staining buffer. Cells were washed and incubated for 30 minutes with anti-EpCAM and and CA 19-9 conjugated with Alexa488 and PerCP, respectively.

Isotype controls were performed for each sentinel node stained, exemplified by the staining of of SN1 from patient 5 (figure 8 A).

Analysis was performed by acquiring 100.000 events from each sample using a FACSAria flow cytometer.

Results of the analysis was presented as indicated in figures 8 B and 8 C (patient 1 and 2, respectively).

Results of the analysis is presented in table 2 below:

| Pat no: | Sentinel node no: | EpCAM+ (% of total events) | CA19-9+ (% of total events) |
|---|---|---|---|
| 1 | SN 1 | 1.9 | 5.6 |
| 1 | SN 2 | 0.18 | 0.72 |
| 1 | SN 3 | 0.051 | 0.093 |
| 2 | SN 1 | 1.3 | 0.42 |
| 2 | SN 2 | 0.15 | 0.13 |
| 3 | SN 1 | 0.038 | 0.042 |
| 4 | SN 1 | 0.088 | 0.024 |
| 4 | SN 2 | 2.8 | 24.7 |
| 5 | SN 1 | 0.79 | 1.7 |
| 5 | SN 2 | 1.3 | 2.4 |
| 6 | SN 1 | 0.26 | 0.078 |
| 6 | SN 2 | 0.55 | 0.27 |
| 7 | SN 1 | 0.069 | 1.3 |
| 7 | SN 2 | 0.064 | 0.65 |

Data such as that presented in the table above could be used for finding of optimal cut-off values for each marker and markers in combination. In order to find reliable cut-off values, however, the sample size would need to be much larger.

In case the true status of each sample was known, sensitivity and specificity for different cut-off values could easily be calculated. An optimal cut-off value for each population could then be generated using, e.g. ROC curves, as presented above.

In case the true status is unknown, the prevalence of colon cancer cells in sentinel lymph nodes in a relevant patient population would have to be known. Based on the test results for each node, each sample would be categorised into a assumed negative group and a assumed positive group where the proportional size of each group would match the prevalence in the relevant patient population. Reference intervals would then be calculated for both groups.

If the data generated conforms to a distributional function such as, e.g., the Normal distribution, the reference intervals can be calculated using a parametric method. Using parametric methods is generally preferred since confidence intervals of reference intervals is generally narrower unless the sample size is very large.

If the data generated does not conform to any distributional function, a non-parametric method would have to be use. In order to reduce the confidence interval a very large sample size would be needed.

Comparing of the reference intervals for both groups of samples (assumed positive and assumed negative) can be used to set an appropriate cut-off value. The cut-off value could be set, e.g., to minimise the overlap of the intervals or to minimise the risk of a false-negative diagnosis.

**Example 5**

**Intracellular staining for CK20 in a colon cancer cell line. Reference experiment.**

The standard approach for detection of metastases in lymph nodes from colon cancer is immunohistochemical staining using CK20 antibodies. In order to evaluate this marker for detection of metastases by flow cytometry intracellular staining of CK20 and detection with an APC-conjugated secondary antibody was performed.
For initial testing decreasing numbers of the DLD-1 colon cancer cell line were added to PBMCs. Starting with a concentration of 9% added tumour cells it was determined that all CK20 positive cells were detected (Fig. 9A, top panel). Using a three-fold dilution series it was possible to detect CK20 positive tumour cells down to a concentration of 0.037% added cells (Fig. 9A). The background staining in PBMC without the addition of DLD-1 cells was 0.009% CK20 positive cells (Fig. 9A, bottom panel), and the isotype control gave a non-specific staining with an average of 0.011% positive cells (data not shown), indicating a low number of false positive events. CK20 positive cells have been described in PBMCs from healthy individuals, suggesting a normal transient existence of cytokeratin positive epithelial cells in the blood. When we intracellularly stained PBMCs for CK20 we were able to detect 0.19% and 0.16% CK20 positive cells as exemplified in a colon cancer patient with metastatic disease and a healthy individual, respectively (Fig. 9B, left panels). In contrast, 0.03% and 0.02% positivity was detected when an isotype control was used (Fig. 9B, right panels). Thus CK20 positive epithelial cells even seem to be present in the blood at a low level in healthy individuals

**Example 6**

**Staining of surface markers on a colon cancer cell line**

Since CK20 positive cells were identified at low levels even in healthy individuals and intracellular staining is more cumbersome, the usefulness of directly conjugated antibodies recognizing colon cancer cell surface makes was investigated. The dilution series of DLD-1 supplemented tumour cells was therefore stained with antibodies specific for the two tumour-associated surface antigens EpCAM and CA19-9, conjugated with the fluorophores AF488 and PerCP, respectively.
EpCAM was expressed on almost all the DLD-1 colon cancer cells (Fig. 9A). The number of detected cells correlates well with the staining for CK20. The detection of the lowest number of tumour cells added (0.012% DLD-1 cells) was identified as 0.014% EpCAM positive cells (Fig. 9A). Since the background staining using PBMCs alone was 0.004% detected events, we conclude that EpCAM positive cells can be detected with good accuracy within small samples of cells.
The antigen CA19-9 is expressed on approximately 80% of the DLD-1 tumour cells (Fig. 10B). The average ratio between CA19-9$^+$ and the added number of cells for the 5 highest concentrations was 0.80 (range 0.73-0 91). The background staining in PBMCs with no added colon cancer cells was slightly higher for CA19-9 than for EpCAM, with 0.017% cells being CA19-9 positive (Fig. 10B. bottom panel), compared to 0.004% being EpCAM positive (Fig. 10A, bottom panel). The presence of tumour cells was readily detectable at as low concentrations as 0.11% added cells without interfering with background staining. So even though the background is slightly higher than that for staining with EpCAM, CA19-9 appears to be a useful marker for detection of colon cancer cells by flow cytometry.
Co-expression of the two tumour-associated markers was evaluated by analysis of EpCAM versus CA19-9 expression, using dot plots with log green fluorescence (AF488) versus log red fluorescence (PerCP). There was a good correlation with the number of detected cells from 9% down to 0.11% added cells (Fig. 10C). On average, 84% of the added cells were double positive for EpCAM and CA19-9 expression (Fig. 10C, upper right quadrant), in concordance with single staining data whereby CA19-9 was expressed on 80% of the cells (Fig. 10B). An average of 24% of the cells was EpCAM$^+$CA19-9$^-$ in the dilution series (Fig. 10C, lower right quadrants). The numbers of CA19-9 single positive cells were very low (average 0.5%) (Fig. 10C, upper left quadrant). Thus the vast majority of CA19-9$^+$ DLD-1 cells concurrently express EpCAM. The use of fluorescence on both axes increased the background staining in PBMCs to 0.087% (Fig. 10C, bottom panel). As most of these events (0.079%) were confined to the upper left quadrant, the increased background is due to a higher number of false positive CA19-9 events. Neither of the isotype controls, (IgG2a AF488 or IgG1 PerCP) gave any significant non-specific staining (data not included). Detection of tumour cells in PBMCs spiked with the DLD-1 colon cancer cell line can therefore be facilitated by staining for multiple cell surface markers.

**Example 7**

*Detection of tumour cells in sentinel nodes*

To verify the usefulness of the directly conjugated antibodies specific for EpCAM and CA19-9 in patients with colon cancer, freshly retrieved single cell suspensions from 14 sentinel nodes from 7 patients were analysed (Table 4). The cells were intracellularly stained in parallel for CK20 to allow comparison. Isotype controls for cytokeratin 20, EpCAM and CA19-9 were used to determine the presence of positive cells (Fig 13A). in average, 0.32, 0.21 and 0.16% cells were stained positive when isotype controls for CK20, EpCAM and CA19-9 were used, respectively.

In patient 1 three sentinel nodes were identified (SN1-3). In SN1 there was a clear presence of metastatic colon cancer cells, of which 5.6% were CK20 positive and the same fraction of cells were identified as being CA19-9 positive (Fig. 13A). EpCAM appeared to be expressed on a third (34%) of the metastatic cells (or 1.9% of total events). Metastatic cells were also present in SN2, 0.57% of the cells being CK20 positive and 0.72% being positive for CA19-9. EpCAM expression was 0.18%, which in comparison to the cytokeratin expression represents 32% of the metastatic cells, and 25% compared to the CA19-9 positive cells. However, the number of detected EpCAM positive cells are close to the Isotype control level. The investigation of SN3 revealed only very few events positive for CK20, EpCAM or CA19-9, close to the detection levels determined by isotype controls and thus SN3 was considered as being negative for the presence of metastatic colon cancer cells.

Two sentinel nodes were identified intraoperatively In patient 2 (Fig 13B). In SN1 CK20 and CA19-9 stainings were comparable with 0.69% and 0.42% of detected metastatic cells, whereas 1.3% of the detected events were EpCAM$^+$. Again in SN2 the detected number of positive events using all three markers was close to detection limit, as determined by isotype controls, and SN2 was thus considered free from metastases. Compared to the test system with PBMCs spiked with tumour cells (Fig 10), samples of lymph node tissue were less homogenous, resulting in a higher background.

*Discussion*

We here present a novel multiplex method for the rapid and reproducible detection of metastatic cells In lymph nodes. Hitherto, detection of metastases is based on the microscopical investigation of a few sections from lymph node material evaluated by a pathologist The method is time consuming, labour intensive, costly and subjective to the individual pathologist's evaluation. For example the pressure on the pathologist to evaluate a frozen section taken from the operating room, with a surgeon awaiting the verdict whether or not to continue and expanding the surgical procedure, causes a situation with the risk of false positive results. Moreover, the procedure only permits the analyses of a few 6 $\mu$m sections creating a situation where false negative results are likely since parts of the lymph node has not even been investigated. To bypass this obstacles and to permit a rapid, objective method for the evaluation of metastatic cells in lymph nodes we have set up a system investigating a statistically relevant sample of the whole lymph node,where the presence of metastatic cells is reliably identified by multiplex staining of cell surface molecules present on colon cells (Figure 6), cells that under normal conditions are not present in the lymph node. The combination of at least four markers increases the sensitivity and specificity of the assay by decreasing the number of false positive and false negative samples. Thus, the results from the investigation will have increased positive and negative predictive values compared to microscopical evaluation, allowing the surgeon to more reliable plan the extent of surgery from the lymph node investigation. Moreover, since the extraction of single cells from lymph nodes is an easy and efficient procedure, and that antibodies with high affinities and high binding rates ($k_{on}$) are used, the binding of detection reagents are rapid permitting short incubation times and an objective readout within a few minutes by flow cytometry. The use of flow cytometry for analyses permits a higher degree of standardisation and simplifies the quality control work by the use of standards.

**References**

Cabanas RM. An approach for the treatment of penile carcinoma. Cancer 1977;39(2):456-66.

Morton D, Essner R, Hoon DS, at al. Long-Term Results of Lymphatic Mapping and Sentinel Lymphadenectomy for Early-Stage Melanoma: Implications of Nodal Microanatomy and Molecular Staging on Detection of Nodal Micrometastasis. American Surgical Association 123rd Annual Meeting. Washington, DC: American Surgical Association, 2003:60.

Merrie AEH, Yun K, Gunn J, Phillips LV, McCall JL: Analysis of potential markers for detection of submicrosoopic lymph node metastases in breast cancer. Br J Cancer 1999:80:2019-2024.

Noguchi M, Tsugawa K, Bando E, Kawahara F, Miwa K, Yokoyama K, Nakajima K, Tonami N: Sentinel lymphadenectomy In breast cancer; Identification of sentinel lymph node and detection of metastases. Breast Cancer Res Treat 1999;53-197-104.

# EP 2 472 264 B1

M P G Leers, R H M G Schoffelen, J G M Hoop, P H M H Theunissen, J W A Oosterhuis, H vd Bijl, A Rahmy, W Tan, M Nap: Multiparameter flow cytometry as a tool for the detection of micrometastatic tumour cells in the sentinel lymph node procedure of patients with breast cancer. J Clin Pathol 2002;55:359-366.

Donald L. Weaver. David N. Krag, Edward A. Manna, Takamaru Ashikaga, Brenda L. Waters, Seth P. Harlow, Kenneth D. Bauer, Thomas B. Julian: Detection of occult sentinel lymph node micrometastases by immunohistochemistry in breast cancer. Cancer. 2006 Aug 15;107(4):661-7.

## Claims

1. A one step method for direct and simultaneous detection of a cancer cell or cancer cell element in a biological sample, namely tumour cell detection in sentinel nodes, metinel nodes, or other lymph nodes, the method comprising

    i) adding to said biological sample a panel of labelled agents, which bind to three or more different extracellular markers, wherein at least one of the agents is cancer specific and wherein the panel contains:

        - one or more agents binding to cancer-type specific extracellular markers, wherein the cancer-type specific marker is CA19-9, and
        - one or more agents binding to tissue-specific extracellular markers that are general epithelial extracellular markers, wherein the general epithelial marker is Ep-CAM;

    ii) subjecting the thus obtained biological sample to flow cytometry.

2. A method according to claim 1, wherein the labelled agents bind to four or more different extracellular markers.

3. A method according to claim 1 or 2, wherein the agents are monoclonal antibodies or fragments thereof.

4. A method according to any of the preceding claims, wherein the cancer cells are detected with a sensitivity of 90% or more, such as, e.g., 91% or more, 92% or more, 93% or more, 94% or more, 95% or more, 96% or more, 97% or more, 98% or more, 99% or more, or 100%.

5. A method according to any of the preceding claims, wherein the cancer cells are detected with a specificity of 95% or more, such as, e.g., 96% or more, 97% or more, 98% or more, 99% or more, or 100% meaning that 95% or more, such as, e.g., 96% or more, 97% or more, 98% or more, 99% or more, or 100% of the sentinel or metinel lymph nodes being free of cancer cells will be correctly diagnosed as negative.

## Patentansprüche

1. Einstufiges Verfahren zum direkten und gleichzeitigen Nachweis von Krebszellen oder Krebszellbestandteilen in einer biologischen Probe, nämlich Nachweis von Tumorzellen in Wächterlymphknoten, Metinel-Lymphknoten oder anderen Lymphknoten, wobei die Methode umfasst:

    i) Zugabe einer Auswahl gelabelter Stoffe zu der biologischen Probe, die an drei oder mehr verschiedene extrazelluläre Marker binden, wobei mindestens einer der Stoffe krebsspezifisch ist und wobei die Auswahl enthält:

        - einen oder mehrere Stoffe, die an krebsartspezifische extrazelluläre Marker binden, wobei der krebsartspezifische Marker CA 19-9 ist, und
        - einen oder mehrere Stoffe, die an gewebespezifische extrazelluläre Marker binden, die allgemeine epitheliale extrazelluläre Marker sind, wobei der allgemeine epitheliale Marker Ep-CAM ist;

    ii) Zufuhr der hierdurch erhaltenen biologischen Probe zur Durchflusszytometrie.

2. Verfahren nach Anspruch 1, wobei die gelabelten Stoffe an vier oder mehr verschiedene extrazelluläre Marker binden.

**3.** Verfahren nach Anspruch 1 oder 2, wobei die Stoffe monoklonale Antiköper oder Fragmente davon sind.

**4.** Verfahren nach einem der vorstehenden Ansprüche, wobei die Krebszellen mit einer Sensitivität von 90% oder mehr nachgewiesen werden, wie beispielsweise 91% oder mehr, 92% oder mehr, 93% oder mehr, 94% oder mehr, 95% oder mehr, 96% oder mehr, 97% oder mehr, 98% oder mehr, 99% oder mehr, oder 100%.

**5.** Verfahren nach einem der vorstehenden Ansprüche, wobei die Krebszellen mit einer Spezifität von 95% oder mehr nachgewiesen werden, wie beispielsweise 96% oder mehr, 97% oder mehr, 98% oder mehr, 99% oder mehr, oder 100%, was bedeutet, dass 95% oder mehr, 96% oder mehr, 97% oder mehr, 98% oder mehr, 99% oder mehr, oder 100% der Wächter- oder Metinel-Lymphknoten, die frei von Krebszellen sind, korrekt als negativ diagnostiziert werden.

## Revendications

**1.** Méthode à une étape pour la détection directe et simultanée d'une cellule cancéreuse ou d'un élément de cellule cancéreuse dans un échantillon biologique, à savoir la détection de cellules de tumeur dans des ganglions sentinelles, ganglions métinelles ou autres ganglions lymphatiques, la méthode comprenant

  i) ajouter audit échantillon biologique un panneau d'agents marqués qui se lient à trois ou plusieurs marqueurs extracellulaires différents, où au moins un des agents est spécifique au cancer et où le panneau continent :

  - un ou plusieurs agents se liant à des marqueurs extracellulaires spécifiques du type de cancer, où le marqueur spécifique du type de cancer est CA19-9, et
  - un ou plusieurs agents se liant à des marqueurs extracellulaires spécifiques au tissu qui sont des marqueurs extracellulaires épithéliaux généraux, où le marqueur épithélial général est Ep-CAM ;

  ii) soumettre l'échantillon biologique ainsi obtenu à une cytométrie en flux.

**2.** Méthode selon la revendication 1, où les agents marqués se lient à quatre ou plus de marqueurs extracellulaires différents.

**3.** Méthode selon la revendication 1 ou 2, où les agents sont des anticorps monoclonaux ou des fragments de ceux-ci.

**4.** Méthode selon l'une quelconque des revendications précédentes, où les cellules cancéreuses sont détectées avec une sensibilité de 90% ou plus, comme par exemple 91% ou plus, 92% ou plus, 93% ou plus, 94% ou plus, 95% ou plus, 96% ou plus, 97% ou plus, 98% ou plus, 99% ou plus ou 100%.

**5.** Méthode selon l'une quelconque des revendications précédentes, où les cellules cancéreuses sont détectées avec une spécificité de 95% ou plus, comme par exemple 96% ou plus, 97% ou plus, 98% ou plus, 99% ou plus ou 100% ce qui signifie que 95% ou plus, comme par exemple 96% ou plus, 97% ou plus, 98% ou plus, 99% ou plus ou 100% des ganglions lymphatiques sentinelles ou métinelles exemptes de cellules cancéreuses seront correctement diagnostiquées comme étant négatifs.

## CA-19-9

3% CCL221

1% CCL221

).33% CCL221

0% CCL221

## Fig. 1

# Epcam-like

Fig. 2

Fig. 3

Fig. 4

Fig. 5

Fig. 6

28

A

B

Fig. 7

Fig. 8 A

Fig. 8 B

Fig. 8 C

Fig. 9A

Fig. 9B

Fig. 10

**A**

EpCAM

$y = 0.9751x - 0.0002$
$R^2 = 0.9988$

Tumour cells detected test 2 (%)

Tumour cells detected test 1 (%)

**B**

CA19-9

$y = 0.6976x + 0.003$
$R^2 = 0.9831$

Tumour cells detected test 2 (%)

Tumour cells detected test 1 (%)

**C**

EpCam and CA19-9

$y = 0.9521x + 0.0001$
$R^2 = 0.9985$

Tumour cells detected test 2 (%)

Tumour cells detected test 1 (%)

## Fig. 11

**Fig. 12**

Fig. 13

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 9828622 A1

## Non-patent literature cited in the description

- **CABANAS RM.** An approach for the treatment of penile carcinoma. *Cancer,* 1977, vol. 39 (2), 456-66
- Long-Term Results of Lymphatic Mapping and Sentinel Lymphadenectomy for Early-Stage Melanoma: Implications of Nodal Microanatomy and Molecular Staging on Detection of Nodal Micrometastasis. **MORTON D ; ESSNER R ; HOON DS.** American Surgical Association 123rd Annual Meeting. American Surgical Association, 2003, 60
- **MERRIE AEH ; YUN K ; GUNN J ; PHILLIPS LV ; MCCALL JL.** Analysis of potential markers for detection of submicrosoopic lymph node metastases in breast cancer. *Br J Cancer,* 1999, vol. 80, 2019-2024
- **NOGUCHI M ; TSUGAWA K ; BANDO E ; KAWAHARA F ; MIWA K ; YOKOYAMA K ; NAKAJIMA K ; TONAMI N.** Sentinel lymphadenectomy In breast cancer; Identification of sentinel lymph node and detection of metastases. *Breast Cancer Res Treat,* 1999, vol. 53, 197-104
- **M P G LEERS ; R H M G SCHOFFELEN ; J G M HOOP ; P H M H THEUNISSEN ; J W A OOSTERHUIS ; H VD BIJL ; A RAHMY ; W TAN ; M NAP.** Multiparameter flow cytometry as a tool for the detection of micrometastatic tumour cells in the sentinel lymph node procedure of patients with breast cancer. *J Clin Pathol,* 2002, vol. 55, 359-366
- **DONALD L. WEAVER ; DAVID N. KRAG ; EDWARD A. MANNA ; TAKAMARU ASHIKAGA ; BRENDA L. WATERS ; SETH P. HARLOW ; KENNETH D. BAUER ; THOMAS B. JULIAN.** Detection of occult sentinel lymph node micrometastases by immunohistochemistry in breast cancer. *Cancer,* 15 August 2006, vol. 107 (4), 661-7